# EUROPEAN PATENT APPLICATION

(11) **EP 4 115 740 A1**
(43) Date of publication of application: **11.01.2023**
(21) Application number: 21764266.9
(22) Date of filing: 04.03.2021
(51) Int. Cl.: A23C 9/127, A23C 19/04, A23L 5/00, C12N 1/19, C12N 1/21, C12N 15/54, C12N 9/10

(54) **MUTANT TRANSGLUTAMINASE**

(30) Priority: 04.03.2020 JP 2020036788
(71) Applicant: AJINOMOTO CO., INC., Chuo-ku Tokyo 104-8315 (JP)
(72) Inventor: KOBAYASHI, Hiroaki, Kawasaki-shi, Kanagawa 210-8681 (JP); KOENUMA, Keiko, Kawasaki-shi, Kanagawa 210-8681 (JP); NOZAKI, Hiroyuki, Kawasaki-shi, Kanagawa 210-8681 (JP); YOKOYAMA, Noriko, Kawasaki-shi, Kanagawa 210-8681 (JP); ONO, Takuto, Kawasaki-shi, Kanagawa 210-8681 (JP)
(74) Representative: Strehl Schübel-Hopf & Partner
(86) International application number: PCT/JP2021/008341
(87) International publication number: WO 2021/177392

(57) **Abstract**

A mutant TG having low acid tolerance is provided. A mutant TG having a mutation at amino acid residue(s) such as A261, V271, A322, V326, D46, M16, Y34, W38, and S199.

## Description

### Technical Field

The present invention relates to a mutant transglutaminase (mutant TG) having low acid tolerance and use of the same.

### Background Art

Transglutaminase (TG) is known as an enzyme that catalyzes cross-linking of proteins, and has been used for improving physical properties of foods. For example, a method for producing yogurt using TG has been known (Patent document 1).

Various mutant TGs having modified functions have also been known. As the mutant TGs include, for example, mutant TGs having properties such as lowered heat tolerance, improved heat tolerance, and improved oxidation tolerance (Patent document 2), mutant TGs having improved heat tolerance (Patent document 3), mutant TGs having improved heat tolerance and/or improved pH stability (Patent document 4), and mutant TGs having improved specific activity (Patent document 5) have been known. As mutations for improving heat tolerance and/or pH stability of TGs, specifically, for example, mutations of replacing an amino acid residue such as D46 to a cysteine residue to thereby introduce a disulfide bond have been known (Patent document 4). As mutations for improving specific activity of TGs, specifically, for example, mutations at M16 such as M16T, mutations at Y34 such as Y34F, mutations at S199 such as S199A, and mutations at W38 such as W38F (Patent document 5).

However, there have not been known mutations for decreasing acid tolerance of TGs.

### Prior art references

### Patent documents

Patent document 1: JPH6-197688A
Patent document 2: WO2019/107288A1
Patent document 3: WO2010/101256A1
Patent document 4: WO2008/099898A1
Patent document 5: JP2008-194004A

### Summary of Invention

### Object to be Achieved by the Invention

An object of the present invention is to provide a mutant TG having low acid tolerance.

### Means for Achieving the Object

The inventors of the present invention found mutations for decreasing acid tolerance of TGs and accomplished the present invention.

The present invention can be thus embodied as follows.
[1] A method for producing a food, the method comprising:
   a step of treating a food raw material with a mutant transglutaminase,
   wherein the food is a food (A) or (B) shown below:
      (A) a food whose production is accompanied by a decrease in pH;
      (B) a food containing the food (A),
   wherein the mutant transglutaminase is a protein having a specific mutation in the amino acid sequence of a wild-type transglutaminase, and having a transglutaminase activity, and
   wherein the specific mutation is a mutation for decreasing acid tolerance.
[2] The method mentioned above, wherein the residual activity after treating the mutant transglutaminase at pH4.0, 37°C for one hour is 80% or less in term of the relative value to the residual activity after treating the mutant transglutaminase at pH6.0, 37°C for one hour which is taken as 100%.
[3] The method mentioned above, wherein the residual activity after treating the mutant transglutaminase at pH4.0, 37°C for one hour is 50% or less in term of the relative value to the residual activity after treating the mutant transglutaminase at pH6.0, 37°C for one hour which is taken as 100%.
[4] The method mentioned above, wherein the specific mutation is mutation(s) at one or more amino acid residues selected from the followings:
   A261, V271, A322, V326, D46, M16, Y34, W38, and S199.
[5] The method mentioned above, wherein the specific mutation comprises mutation(s) at one or more amino acid residues selected from the followings:
   A261, V271, A322, V326, and D46.
[6] The method mentioned above, wherein the specific mutation comprises one or more mutations selected from the followings:
   A261C, V271C, A322C, V326C, D46P, M16T, Y34F, W38F, and S199A.
[7] The method mentioned above, wherein the specific mutation comprises one or more mutations selected from the followings:
   A261C, V271C, A322C, V326C, and D46P.
[8] The method mentioned above, wherein the specific mutation comprises any of the following mutations:
   A261C/A322C, A261C/A322C/S199A, V271C/V326C, M16T/Y34F, and M16T/S 199A.
[9] The method mentioned above, wherein the wild-type transglutaminase is a protein having an amino acid sequence of a mature transglutaminase of a *Streptomyces* bacterium.
[10] The method mentioned above, wherein the *Streptomyces* bacterium is *Streptomyces mobaraensis.*
[11] The method mentioned above, wherein the wild-type transglutaminase is any of the following proteins:
   (a) a protein comprising the amino acid sequence of SEQ ID NO: 2;
   (b) a protein comprising the amino acid sequence of SEQ ID NO: 2 but including substitution, deletion, insertion, and/or addition of 1 to 10 amino acid residues; and
   (c) a protein comprising an amino acid sequence having an identity of 90% or higher to the amino acid sequence of SEQ ID NO: 2.
[12] The method mentioned above, wherein the food (A) is yogurt or cheese, and wherein the food raw material is a milk raw material.
[13] The method mentioned above, wherein the food (B) is ice cream, the ice cream containing yogurt or cheese.
[14] The method mentioned above, wherein the mutant transglutaminase is added to the food raw material at a time point when the pH of the food raw material is 5.0 or higher.
[15] A composition for producing a food, the composition comprising a mutant transglutaminase,
   wherein the food is a food (A) or (B) shown below:
      (A) a food whose production is accompanied by a decrease in pH;
      (B) a food containing the food (A),
   wherein the mutant transglutaminase is a protein having a specific mutation in the amino acid sequence of a wild-type transglutaminase, and having a transglutaminase activity, and
   wherein the specific mutation is a mutation for decreasing acid tolerance.
[16] The composition mentioned above, wherein the residual activity after treating the mutant transglutaminase at pH4.0, 37°C for one hour is 80% or less in term of the relative value to the residual activity after treating the mutant transglutaminase at pH6.0, 37°C for one hour which is taken as 100%.
[17] The composition mentioned above, wherein the residual activity after treating the mutant transglutaminase at pH4.0, 37°C for one hour is 50% or less in term of the relative value to the residual activity after treating the mutant transglutaminase at pH6.0, 37°C for one hour which is taken as 100%.
[18] The composition mentioned above, wherein the specific mutation is mutation(s) at one or more amino acid residues selected from the followings:
   A261, V271, A322, V326, D46, M16, Y34, W38, and S199.
[19] The composition mentioned above, wherein the specific mutation comprises mutation(s) at one or more amino acid residues selected from the followings:
   A261, V271, A322, V326, and D46.
[20] The composition mentioned above, wherein the specific mutation comprises one or more mutations selected from the followings:
   A261C, V271C, A322C, V326C, D46P, M16T, Y34F, W38F, and S199A.
[21] The composition mentioned above, wherein the specific mutation comprises one or more mutations selected from the followings:
   A261C, V271C, A322C, V326C, and D46P.
[22] The composition mentioned above, wherein the specific mutation comprises any of the following mutations:
   A261C/A322C, A261C/A322C/S199A, V271C/V326C, M16T/Y34F, and M16T/S 199A.
[23] The composition mentioned above, wherein the wild-type transglutaminase is a protein having an amino acid sequence of a mature transglutaminase of a *Streptomyces* bacterium.
[24] The composition mentioned above, wherein the *Streptomyces* bacterium is *Streptomyces mobaraensis.*
[25] The composition mentioned above, wherein the wild-type transglutaminase is any of the following proteins:
   (a) a protein comprising the amino acid sequence of SEQ ID NO: 2;
   (b) a protein comprising the amino acid sequence of SEQ ID NO: 2 but including substitution, deletion, insertion, and/or addition of 1 to 10 amino acid residues; and
   (c) a protein comprising an amino acid sequence having an identity of 90% or higher to the amino acid sequence of SEQ ID NO: 2.
[26] The composition mentioned above, wherein the food (A) is yogurt or cheese, and wherein the food raw material is a milk raw material.
[27] The composition mentioned above, wherein the food (B) is ice cream, the ice cream containing yogurt or cheese.
[28] A mutant transglutaminase, having a specific mutation in the amino acid sequence of a wild-type transglutaminase, and having a transglutaminase activity,
   wherein the specific mutation is a mutation for decreasing acid tolerance, and
   wherein the specific mutation is mutation(s) at one or more amino acid residues selected from the followings:
      A261, V271, A322, V326, D46, M16, Y34, W38, and S199,
   provided that none of the mutations at M16, Y34, W38, and S199 is selected solely, and
   provided that the mutation at D46 is a mutation other than D46C when the mutation at D46 is selected solely.
[29] The mutant transglutaminase mentioned above, wherein the residual activity after treating the mutant transglutaminase at pH4.0, 37°C for one hour is 80% or less in term of the relative value to the residual activity after treating the mutant transglutaminase at pH6.0, 37°C for one hour which is taken as 100%.
[30] The mutant transglutaminase mentioned above, wherein the residual activity after treating the mutant transglutaminase at pH4.0, 37°C for one hour is 50% or less in term of the relative value to the residual activity after treating the mutant transglutaminase at pH6.0, 37°C for one hour which is taken as 100%.
[31] The mutant transglutaminase mentioned above, wherein the specific mutation comprises mutation(s) at one or more amino acid residues selected from the followings:
   A261, V271, A322, V326, and D46.
[32] The mutant transglutaminase mentioned above, wherein the specific mutation comprises one or more mutations selected from the followings:
   A261C, V271C, A322C, V326C, D46P, M16T, Y34F, W38F, and S199A.
[33] The mutant transglutaminase mentioned above, wherein the specific mutation comprises one or more mutations selected from the followings:
   A261C, V271C, A322C, V326C, and D46P.
[34] The mutant transglutaminase mentioned above, wherein the specific mutation comprises any of the following mutations:
   A261C/A322C, A261C/A322C/S199A, V271C/V326C, M16T/Y34F, and M16T/S199A.
[35] The mutant transglutaminase mentioned above, wherein the wild-type transglutaminase is a protein having an amino acid sequence of a mature transglutaminase of a *Streptomyces* bacterium.
[36] The mutant transglutaminase mentioned above, wherein the *Streptomyces* bacterium is *Streptomyces mobaraensis.*
[37] The mutant transglutaminase mentioned above, wherein the wild-type transglutaminase is any of the following proteins:
   (a) a protein comprising the amino acid sequence of SEQ ID NO: 2;
   (b) a protein comprising the amino acid sequence of SEQ ID NO: 2 but including substitution, deletion, insertion, and/or addition of 1 to 10 amino acid residues; and
   (c) a protein comprising an amino acid sequence having an identity of 90% or higher to the amino acid sequence of SEQ ID NO: 2.
[38] A gene encoding the mutant transglutaminase mentioned above.
[39] A vector comprising the gene mentioned above.
[40] A microorganism having the gene mentioned above.
[41] The microorganism mentioned above, which is a bacterium or a yeast.
[42] The microorganism mentioned above, which is a coryneform bacterium or a bacterium of the family *Enterobacteriaceae.*
[43] The microorganism mentioned above, which is a *Corynebacterium* bacterium or an *Escherichia* bacterium.
[44] The microorganism mentioned above, which is *Corynebacterium glutamicum* or *Escherichia coli.*

### Brief Description of Drawings

[FIG. 1] A diagram showing the breaking strength of yogurt produced by using a wild-type TG or each mutant TG.
[FIG. 2] A diagram showing the TG activity (absorbance by hydroxamate method) in a supernatant of yogurt produced by using a wild-type TG or each mutant TG.
[FIG. 3] A diagram showing the change over time of the TG activity (fluorescence intensity by fluorescence method) upon producing yogurt using a wild-type TG or a mutant TG (A261C/A322C).
[FIG. 4] A diagram showing the change over time of the pH and the TG activity (absorbance by hydroxamate method) upon producing yogurt using a wild-type TG or a mutant TG (A261C/A322C).

### Modes for Carrying out the Invention

### <1> Mutant transglutaminase (mutant TG)

The present invention provides a transglutaminase (TG) having a "specific mutation" as described herein.

The term "transglutaminase (TG)" may refer to a protein having an activity of catalyzing an acyl group transfer reaction between an amide group of a glutamine residue in a protein and a primary amine (EC 2.3.2.13, etc.). This activity is also referred to as "TG activity". A gene encoding TG is also referred to as a "TG gene". Examples of the primary amine include a lysine residue in a protein. That is, the term "TG activity" may specifically refer to an activity of catalyzing a cross-linking reaction between a glutamine residue in a protein and a lysine residue in a protein. The cross-linking reaction may result in formation of an intramolecular cross-link and/or an intermolecular cross-link. The cross-linking reaction may typically result in formation of at least the intermolecular cross-link.

The TG activity can be measured, for example, by the hydroxamate method (Lorand,L., et al.: Anal. Biochem., 44, 221-213(1971)) or by the fluorescence method (Takagi,J., et al.: Anal. Biochem., 153, 296-298(1986)). The term "TG activity" may refer to the TG activity measured by the hydroxamate method, unless otherwise stated.

The procedure for measuring the TG activity by the hydroxamate method is as follows. That is, the TG activity can be determined by incubating the enzyme with the substrate (i.e., benzyloxycarbonyl-L-glutaminylglycine and hydroxylamine) at 37°C, pH 6.0 and measuring enzyme- and substrate-dependent formation of hydroxamic acid. The formation of hydroxamic acid can be measured by forming an iron complex of hydroxamic acid in the presence of trichloroacetic acid and measuring an increase in absorbance at 525 nm as an indicator. An amount of enzyme that catalyzes the formation of 1 µmol of hydroxamic acid per minute under the aforementioned conditions is defined as 1 U (unit).

The procedure for measuring the TG activity by the fluorescence method is as follows. That is, the TG activity can be determined by incubating the enzyme with substrates (i.e., dimethylated casein and monodansylcadaverine (MDC)) at 37°C, pH 7.5 and measuring enzyme- and substrate-dependent incorporation of MDC into dimethylated casein. The incorporation of MDC can be measured by measuring an increase in fluorescence (excitation wavelength 350 nm, emission wavelength 480 nm) as an indicator.

TG having the "specific mutation" is also referred to as "mutant TG". A gene encoding a mutant TG is also referred to as "mutant TG gene".

TG not having the "specific mutation" is also referred to as "wild-type TG". A gene encoding a wild-type TG is also referred to as "wild-type TG gene". The term "wild-type" referred to herein is used for convenience to distinguish the "wild-type" TG from the "mutant" TG, and the "wild-type" one is not limited to those obtained as natural products, provided that it does not have the "specific mutation". The phrase "TG does not have the "specific mutation"" may mean that TG does not have a mutation selected as the "specific mutation". The wild-type TG may or may not have a mutation not selected as the "specific mutation", provided that it does not have a mutation selected as the "specific mutation".

When a certain wild-type TG and a certain mutant TG are identical to each other except for the presence or absence of the "specific mutation", this certain wild-type TG is also referred to as "wild-type TG corresponding to a certain mutant TG", and this certain mutant TG is also referred to as "mutant TG corresponding to a certain wild-type TG".

Hereafter, the wild-type TG will be explained.

The wild-type TG may or may not have the TG activity, provided that the mutant TG corresponding to the wild-type TG has the TG activity. The wild-type TG may typically have the TG activity.

Examples of the wild-type TG include TGs of Actinomycetes (Appl. Environ. Microbiol., 2003, 69(1), 358-366). Examples of the Actinomycetes include *Streptomyces* bacteria. Examples of the *Streptomyces* bacteria include *Streptomyces mobaraensis, Streptomyces cinnamoneus,* and *Streptomyces griseocarneus.* The term *"Streptomyces* bacteria" also includes bacteria that had been classified to the genus *Streptoverticillium.* For example, the term *"Streptomyces mobaraensis"* also includes bacteria that had been classified to *Streptoverticillium mobaraense* or *Streptoverticillium ladakanum.* In addition, the term *"Streptomyces cinnamoneus"* also includes bacteria that had been classified to *Streptoverticillium cinnamoneum.* In addition, the term *"Streptomyces griseocarneus"* also includes bacteria that had been classified to *Streptoverticillium griseocarneum.* TG, for example, can be expressed in the form of comprising a pro-structure moiety, and then can become a mature protein by removal of the pro-structure moiety. The mature protein of TG is also referred to as "mature TG". That is, specific examples of TGs of the organisms exemplified above include mature TGs of the organisms exemplified above. The nucleotide sequence of a part of the TG gene of *Streptoverticillium mobaraense,* the part encoding the mature TG, is shown as SEQ ID NO: 1, and the amino acid sequence of the mature TG encoded by this gene is shown as SEQ ID NO: 2. That is, the wild-type TG gene may be, for example, a gene having any of the nucleotide sequences of the TG genes of the organisms exemplified above (e.g., the nucleotide sequence shown as SEQ ID NO: 1). Also, the wild-type TG may be, for example, a protein having any of the amino acid sequences of the TGs of the organisms exemplified above (e.g., the amino acid sequence shown as SEQ ID NO: 2). The expression "a gene or protein has a nucleotide or amino acid sequence" may mean that a gene or protein comprises the nucleotide or amino acid sequence unless otherwise stated, and also include cases where a gene or protein consists of the nucleotide or amino acid sequence.

The wild-type TG gene may also be a variant of any of the wild-type TG genes exemplified above (e.g., the gene having the nucleotide sequence shown as SEQ ID NO: 1), provided that the encoded TG does not have the "specific mutation". Similarly, the wild-type TG may be a variant of any of the wild-type TGs exemplified above (e.g., the protein having the amino acid sequence shown as SEQ ID NO: 2), provided that the variant does not have the "specific mutation". That is, the term "wild-type TG gene" is not limited to the wild-type TG genes exemplified above (e.g., the gene having the nucleotide sequence shown as SEQ ID NO: 1), but may also include variants thereof. Similarly, the term "wild-type TG" is not limited to the wild-type TGs exemplified above (e.g., the protein having the amino acid sequence shown as SEQ ID NO: 2), but may also include variants thereof. A gene specified with the type of organism from which the gene is derived is not limited to genes themselves found in that organism, and shall also include genes having any of the nucleotide sequences of the genes found in that organism and variants thereof. A protein specified with the type of organism from which the protein is derived is not limited to proteins themselves found in that organism, and shall also include proteins having any of the amino acid sequences of the proteins found in that organism and variants thereof. That is, for example, the term "TG of Streptoverticillium bacteria" is not limited to TGs themselves found in Streptoverticillium bacteria, and shall also include proteins having any of the amino acid sequences of the TGs found in Streptoverticillium bacteria and variants thereof. Examples of the variants include, for example, homologues and artificially modified versions of the genes and proteins exemplified above.

Homologues of the wild-type TG gene or homologues of the wild-type TG can be easily obtained from public databases by, for example, BLAST search or FASTA search using any of the nucleotide sequences of the wild-type TG genes exemplified above or any of the amino acid sequences of the wild-type TGs exemplified above as a query sequence. Furthermore, homologues of the wild-type TG gene can be obtained by, for example, PCR using a chromosome of various organisms as the template, and oligonucleotides prepared on the basis of any of the nucleotide sequences of the wild-type TG genes exemplified above as primers.

The wild-type TG gene may be a gene encoding a protein having any of the aforementioned amino acid sequences (e.g., the amino acid sequence shown as SEQ ID NO: 2) including substitution, deletion, insertion, and/or addition of one or several amino acid residues at one or several positions, provided that the encoded TG does not have the "specific mutation". For example, the encoded protein may have an extended or deleted N-terminus and/or C-terminus. Although the number meant by the term "one or several" used above may differ depending on the positions of amino acid residues in the three-dimensional structure of the protein or the types of amino acid residues, specifically, it may be, for example, 1 to 50, 1 to 40, 1 to 30, 1 to 20, 1 to 10, 1 to 5, or 1 to 3.

The aforementioned substitution, deletion, insertion, or addition of one or several amino acid residues each are a conservative mutation that maintains the original function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the organism from which the gene is derived (mutant or variant).

Furthermore, the wild-type TG gene may be a gene encoding a protein having an amino acid sequence showing an identity of, for example, 50% or more, 65% or more, 80% or more, 90% or more, 95% or more, 97% or more, or 99% or more, to the total amino acid sequence of any of the aforementioned amino acid sequences, provided that the encoded TG does not have the "specific mutation".

Furthermore, the wild-type TG gene may be a gene, such as a DNA, that is able to hybridize under stringent conditions with a probe that can be prepared from any of the aforementioned nucleotide sequences (e.g., the nucleotide sequence shown as SEQ ID NO: 1), such as a sequence complementary to the whole sequence or a partial sequence of any of the aforementioned nucleotide sequences, provided that the encoded TG does not have the "specific mutation". The term "stringent conditions" may refer to conditions under which a so-called specific hybrid is formed, and a non-specific hybrid is not formed. Examples of the stringent conditions include those under which highly identical DNAs hybridize to each other, for example, DNAs not less than 50%, 65%, or 80% identical, preferably not less than 90% identical, more preferably not less than 95% identical, still more preferably not less than 97% identical, particularly preferably not less than 99% identical, hybridize to each other, and DNAs less identical than the above do not hybridize to each other, or conditions of washing of typical Southern hybridization, i.e., conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe used for the aforementioned hybridization may be a part of a sequence that is complementary to the gene as described above. Such a probe can be prepared by PCR using oligonucleotides prepared on the basis of a known gene sequence as primers and a DNA fragment containing any of the aforementioned genes as a template. As the probe, for example, a DNA fragment having a length of about 300 bp can be used. When a DNA fragment having a length of about 300 bp is used as the probe, in particular, the washing conditions of the hybridization may be, for example, 50°C, 2 x SSC and 0.1% SDS.

Furthermore, since properties concerning degeneracy of codons changes depending on a host, the wild-type TG gene may include substitution of respective equivalent codons for any codons. That is, the wild-type TG gene may be a variant of any of the wild-type TG genes exemplified above due to the degeneracy of the genetic code. For example, the wild-type TG gene may be a gene modified so as to have optimal codons according to codon frequencies in a host to be used.

The term "identity" between amino acid sequences means an identity between the amino acid sequences calculated by blastp with default scoring parameters (i.e., Matrix, BLOSUM62; Gap Costs, Existence = 11, Extension = 1; Compositional Adjustments, Conditional compositional score matrix adjustment). The term "identity" between nucleotide sequences means an identity between the nucleotide sequences calculated by blastn with default scoring parameters (i.e., Match/Mismatch Scores = 1, - 2; Gap Costs = Linear).

Hereafter, the mutant TG will be explained.

The mutant TG has the TG activity. The degree of the TG activity of the mutant TG is not particularly limited, so long as the mutant TG can be used for a desired purpose. The TG activity of the mutant TG, for example, may be 50% or more, 60% or more, 70% or more, 80% or more, 90% or more, 100% or more, 120% or more, 150% or more, or 200% or more, may be 10000% or less, 1000% or less, 200% or less, 150% or less, 120% or less, or 100% or less, or may be within a range defined as a non-contradictory combination thereof, of the TG activity of the wild-type TG corresponding to this mutant TG and/or the TG activity of the wild-type TG consisting of the amino acid sequence shown as SEQ ID NO: 2 in terms of the specific activity.

The mutant TG has the "specific mutation" in the amino acid sequence of the wild-type TG.

That is, the mutant TG may be, for example, a protein having the amino acid sequence shown as SEQ ID NO: 2, but having the "specific mutation". The mutant TG may also be, for example, a protein having the amino acid sequence shown as SEQ ID NO: 2, but having the "specific mutation" and further including substitution, deletion, insertion, and/or addition of one or several amino acid residues at position(s) other than the position(s) of the "specific mutation", and having the TG activity.

Also, in other words, the mutant TG may be a protein having the amino acid sequence identical to that of the wild-type TG, except that the mutant TG has the "specific mutation". That is, the mutant TG may be, for example, a protein having the amino acid sequence shown as SEQ ID NO: 2, except that the mutant TG has the "specific mutation". Also, the mutant TG may be, for example, a protein having the amino acid sequence shown as SEQ ID NO: 2, but including substitution, deletion, insertion, and/or addition of one or several amino acid residues, and having the TG activity, except that the mutant TG has the "specific mutation". Also, the mutant TG may be, for example, a protein having an amino acid sequence showing an identity of 80% or more, preferably 90% or more, more preferably 95% or more, still more preferably 97% or more, or particularly preferably 99% or more to the amino acid sequence of SEQ ID NO: 2, and having the TG activity, except that the mutant TG has the "specific mutation".

The mutant TG may comprise another amino acid sequence in addition to such an amino acid sequence of the mutant TG as exemplified above. That is, the mutant TG may be a fusion protein with another amino acid sequence. Furthermore, the mutant TG may be expressed in the form of comprising another amino acid sequence (i.e., in the form of a fusion protein with another amino acid sequence), and may eventually lose a part or the whole of the other amino acid sequence. The phrase "a mutant TG comprises another amino acid sequence" means that the mutant TG eventually obtained comprises another amino acid sequence, unless otherwise stated. On the other hand, The phrase "a mutant TG is expressed in the form of comprising another amino acid sequence" means that the mutant TG comprises another amino acid sequence at least at the time of expression thereof, unless otherwise stated, and does not necessarily mean that the mutant TG eventually obtained comprises another amino acid sequence. The same shall apply to the wild-type TG. The "another amino acid sequence" is not particularly limited, so long as the mutant TG has the TG activity. The "another amino acid sequence" can be appropriately selected depending on various conditions such as use purpose thereof. Examples of the "another amino acid sequence" include, for example, a peptide tag, a signal peptide (also referred to as "signal sequence"), a pro-structure moiety, and a recognition sequence of a protease. The "another amino acid sequence" may be bound to, for example, either one or both of the N-terminus and C-terminus of the mutant TG. As the "another amino acid sequence", one kind of amino acid sequence may be used, or two or more kinds of amino acid sequences may be used in combination.

Specific examples of the peptide tag include His tag, FLAG tag, GST tag, Myc tag, MBP (maltose binding protein), CBP (cellulose binding protein), TRX (thioredoxin), GFP (green fluorescent protein), HRP (horseradish peroxidase), ALP (alkaline phosphatase), and Fc region of antibody. Examples of the His tag include 6xHis tag. The peptide tag can be utilized for, for example, detection and purification of the expressed mutant TG.

The signal peptide is not particularly limited, so long as it functions in a host in which the mutant TG is expressed. Examples of the signal peptide include a signal peptide that is recognized by the Sec system secretory pathway and a signal peptide recognized by the Tat system secretory pathway. Specific examples of the signal peptide that is recognized by the Sec system secretory pathway include signal peptides of cell surface layer proteins of coryneform bacteria. Specific examples of the signal peptides of cell surface layer proteins of coryneform bacteria include the PS1 signal sequence and the PS2 (CspB) signal sequence of C. *glutamicum* (Japanese Patent Laid-open (Kohyo) No. 6-502548), and the SlpA (CspA) signal sequence of C. stationis (Japanese Patent Laid-open (Kokai) No. 10-108675). Specific examples of the signal peptide that is recognized by the Tat system secretory pathway include the TorA signal sequence of *E. coli,* the SufI signal sequence of *E. coli,* the PhoD signal sequence of *Bacillus subtilis,* the LipA signal sequence of *Bacillus subtilis,* and the IMD signal sequence of *Arthrobacter globiformis* (WO2013/118544). The signal peptide can be used for, for example, secretory production of the mutant TG. When secretory production of the mutant TG is carried out by using the signal peptide, the signal peptide may be cleaved at the time of the secretion, and the mutant TG not having the signal peptide may be secreted out of the cell. That is, it is typically acceptable that the mutant TG eventually obtained does not comprises the signal peptide.

Specific examples of the pro-structure moiety include the pro-structure moiety of each of the wild-type TGs exemplified above. The nucleotide sequence of the TG gene of *Streptoverticillium mobaraense* comprising a part encoding the pro-structure moiety is shown as SEQ ID NO: 3, and the amino acid sequence of TG comprising the pro-structure moiety and encoded by this gene is shown as SEQ ID NO: 4. In the SEQ ID NO: 3, positions 1 to 135 correspond to the part encoding the pro-structure moiety, and position 136 and subsequent positions correspond to the part encoding the mature TG (i.e., SEQ ID NO: 1). In the SEQ ID NO: 4, positions 1 to 45 correspond to the pro-structure moiety, and position 46 and subsequent positions correspond to the mature TG (i.e., SEQ ID NO: 2). The mutant TG, for example, may be expressed in the form of comprising the pro-structure moiety, and then may become a mature protein by removal of the pro-structure moiety. When the mutant TG is expressed in the form of comprising the pro-structure moiety, the mutant TG can be activated by removal of the pro-structure moiety. Hence, it is typically acceptable that the mutant TG eventually obtained does not comprises the pro-structure moiety. Removal of the pro-structure moiety can be carried out by, for example, using a processing enzyme. Examples of the processing enzyme include proteases such as SAM-P45 (Appl. Environ. Microbiol., 2003, 69(1), 358-366) and Alcalase. Furthermore, as described below, when the mutant TG is expressed with a recognition sequence of a protease inserted into the connection part of the pro-structure moiety and the mutant TG, the pro-structure moiety can be removed by using the corresponding protease.

Specific examples of the recognition sequence of a protease include the recognition sequence of the Factor Xa protease and the recognition sequence of the proTEV protease. The recognition sequence of a protease can be used for, for example, cleavage of the expressed mutant TG. Specifically, for example, when the mutant TG is expressed as a fusion protein with the other amino acid sequence such as a peptide tag and a pro-structure moiety, if the recognition sequence of a protease is inserted into the connection part of the mutant TG and the other amino acid sequence, the other amino acid sequence can be cleaved from the expressed mutant TG by using the corresponding protease to obtain the mutant TG not having the other amino acid sequence.

The mutant TG gene is not particularly limited, so long as it encodes such a mutant TG as mentioned above. In the present invention, the term "gene" is not limited to DNA, but may include any polynucleotide, so long as it encodes a target protein. That is, the term "mutant TG gene" may refer to any polynucleotide encoding the mutant TG. The mutant TG gene may be DNA, RNA, or a combination thereof. The mutant TG gene may be single-stranded or double-stranded. The mutant TG gene may be a single-stranded DNA or a single-stranded RNA. The mutant TG gene may be a double-stranded DNA, a double-stranded RNA, or a hybrid strand consisting of a DNA strand and an RNA strand. The mutant TG gene may contain both a DNA residue and an RNA residue in a single polynucleotide chain. When the mutant TG gene contains RNA, the aforementioned descriptions concerning DNA, such as those concerning nucleotide sequences exemplified above, may be applied to RNA with appropriately changing wordings to those for RNA as required. The mode of the mutant TG gene can be chosen according to various conditions such as use mode thereof.

Hereafter, the "specific mutation" will be explained.

The "specific mutation" is a mutation for decreasing acid tolerance. That is, the mutant TG has low acid tolerance. The mutant TG may have lower acid tolerance than the wild-type TG. The mutant TG may have lower acid tolerance than, for example, the wild-type TG corresponding to this mutant TG and/or the wild-type TG consisting of the amino acid sequence shown as SEQ ID NO: 2.

The term "acid tolerance" may refer to tolerance against deactivation under acidic conditions. That is, the phrase "a mutant TG has low acid tolerance" may mean that the degree of deactivation caused by treating the mutant TG under acidic conditions is large, and in other words, may mean that the residual activity after treating the mutant TG under acidic conditions is small. Furthermore, the phrase "a mutant TG has lower acid tolerance than a wild-type TG" may mean that the degree of deactivation caused by treating the mutant TG under acidic conditions is larger than the degree of deactivation caused by treating the wild-type TG under acidic conditions, and in other words, may mean that the residual activity after treating the mutant TG under acidic conditions is smaller than the residual activity after treating the wild-type TG under acidic conditions.

The phrase "a mutant TG has low acid tolerance" may mean that, for example, the residual activity after treating the mutant TG under acidic conditions is 80% or less, 70% or less, 60% or less, 50% or less, 40% or less, 30% or less, 20% or less, 10% or less, 5% or less, or 0%. The phrase "a mutant TG has low acid tolerance" may also mean that, for example, the residual activity after treating the mutant TG under acidic conditions is 80% or less, 70% or less, 60% or less, 50% or less, 40% or less, 30% or less, 20% or less, 10% or less, 5% or less, or 0% in term of the relative value to the residual activity after treating the same under control conditions which is taken as 100%.

Examples of the treatment under acidic conditions include treatment at pH5.0, pH4.5, or pH4.0, 37°C for one hour. Examples of the treatment under control conditions include treatment at pH6.0, 37°C for one hour. The phrase "residual activity after treating TG under certain conditions" refers to the ratio of the TG activity after treating TG under the certain conditions to the TG activity before treating TG under the certain conditions. The phrase "treating TG under certain conditions" refers to putting TG under the certain conditions.

For example, the residual activity after treating the mutant TG at pH4.0, 37°C for one hour may be 80% or less in term of the relative value to the residual activity after treating the same at pH6.0, 37°C for one hour which is taken as 100%. Also, for example, the residual activity after treating the mutant TG at pH4.0, 37°C for one hour may be 50% or less in term of the relative value to the residual activity after treating the same at pH6.0, 37°C for one hour which is taken as 100%.

Examples of the "specific mutation" include mutations at the following amino acid residues:
A261, V271, A322, V326, D46, M16, Y34, W38, and S199.

The "specific mutation" may consist of a mutation at one amino acid residue, or may consist of a combination of mutations at two or more amino acid residues. That is, the "specific mutation" may comprise, for example, mutation(s) at one or more amino acid residues selected from these amino acid residues. The "specific mutation", for example, may consist of a mutation at one amino acid residue selected from these amino acid residues, or may consist of a combination of mutations at two or more amino acid residues selected from these amino acid residues.

Mutation at any amino acid residue may or may not be selected solely. Incidentally, for example, it is also acceptable that none of mutations at M16, Y34, W38, and S199 is selected solely.

Particular examples of the "specific mutation" include mutations at A261, V271, A322, V326, and D46. That is, the "specific mutation" may comprise, for example, mutation(s) at one or more amino acid residues selected from A261, V271, A322, V326, and D46. The "specific mutation", for example, may consist of mutation(s) at one or more amino acid residues selected from A261, V271, A322, V326, and D46, or may consist of a combination of mutation(s) at one or more amino acid residues selected from A261, V271, A322, V326, and D46 and mutation(s) at one or more amino acid residues selected from M16, Y34, W38, and S199.

More particular examples of the "specific mutation" include mutations at A322 and D46. That is, the "specific mutation" may comprise, for example, mutation(s) at A322 and/or D46. The "specific mutation", for example, may consist of mutation(s) at A322 and/or D46, or may consist of a combination of mutation(s) at A322 and/or D46 and mutation(s) at one or more amino acid residues selected from A261, V271, V326, M16, Y34, W38, and S199.

In the aforementioned notation used for defining amino acid residues, the numbers represent the positions in the amino acid sequence shown as SEQ ID NO: 2, and the letters at the left side of the numbers represent the amino acid residues at the respective positions in the amino acid sequence shown as SEQ ID NO: 2 (i.e., the amino acid residues before modification at the respective positions). That is, for example, "A261" represents A (Ala) residue at position 261 in the amino acid sequence shown as SEQ ID NO: 2.

In any wild-type TG, each of these amino acid residues represents an "amino acid residue corresponding to each of these amino acid residues in the amino acid sequence shown in SEQ ID NO: 2". That is, for example, "A261" in any wild-type TG represents an amino acid residue corresponding to A (Ala) residue at position 261 in the amino acid sequence shown as SEQ ID NO: 2.

Each of the aforementioned mutations may be substitution of an amino acid residue. In each of the aforementioned mutations, the amino acid residue after modification may be any amino acid residue other than the amino acid residue before modification, provided that acid tolerance of TG is decreased. That is, as the amino acid residue after modification, it is sufficient to select those resulting in a decrease in acid tolerance of TG. Specific examples of the amino acid residue after modification include amino acid residues selected from K (Lys), R (Arg), H (His), A (Ala), V (Val), L (Leu), I (Ile), G (Gly), S (Ser), T (Thr), P (Pro), F (Phe), W (Trp), Y (Tyr), C (Cys), M (Met), D (Asp), E (Glu), N (Asn), and Q (Gln), provided that the amino acid residue after modification is other than the amino acid residue before modification. The amino acid residue after modification at D46 may be selected from, for example, amino acid residues other than C (Cys). The amino acid residue after modification at D46 may be selected from, for example, amino acid residues other than C (Cys) when the "specific mutation" consists of a mutation at D46 (i.e., when a mutation at D46 is selected solely).

Specific examples of the "specific mutation" include the following mutations:
A261C, V271C, A322C, V326C, D46P, M16T, Y34F, W38F, and S199A.

That is, the "specific mutation" may comprise, for example, one or more mutations selected from these mutations. The "specific mutation", for example, may consist of one mutation selected from these mutations, or may consist of a combination of two or more mutations selected from these mutations. The "specific mutation" may also consist of, for example, a combination of one or more mutations selected from these mutations and other mutation(s) at one or more amino acid residues selected from A261, V271, A322, V326, D46, M16, Y34, W38, and S199.

Furthermore, in other words, mutations at A261, V271, A322, V326, D46, M16, Y34, W38, and S199 may be, for example, A261C, V271C, A322C, V326C, D46P, M16T, Y34F, W38F, and S199A, respectively.

Particular examples of the "specific mutation" include A261C, V271C, A322C, V326C, and D46P. That is, the "specific mutation" may comprise, for example, one or more mutations selected from A261C, V271C, A322C, V326C, and D46P. The "specific mutation", for example, may consist of one mutation selected from A261C, V271C, A322C, V326C, and D46P, or may consist of a combination of two or more mutations selected from A261C, V271C, A322C, V326C, and D46P. The "specific mutation" may also consist of, for example, a combination of one or more mutations selected from A261C, V271C, A322C, V326C, and D46P and other mutation(s) at one or more amino acid residues selected from A261, V271, A322, V326, D46, M16, Y34, W38, and S199.

More particular examples of the "specific mutation" include A322C and D46P. That is, the "specific mutation" may comprise, for example, A322C and/or D46P. The "specific mutation" may consist of, for example, A322C and/or D46P. The "specific mutation" may also consist of, for example, a combination of A322C and/or D46P and other mutation(s) at one or more amino acid residues selected from A261, V271, A322, V326, D46, M16, Y34, W38, and S199.

In the aforementioned notation used for defining mutations, the numbers and the letters at the left side of the numbers represent the same as described above. In the aforementioned notation used for defining mutations, the letters at the right side of the numbers represent the amino acid residues after modification at the respective positions. That is, for example, "A261C" represents a mutation of replacing A (Ala) residue at position 261 in the amino acid sequence shown as SEQ ID NO: 2 with C (Cys) residue.

In any wild-type TG, each of these mutations represents a "mutation corresponding to each of these mutations in the amino acid sequence shown in SEQ ID NO: 2". In any wild-type TG, a "mutation corresponding to a mutation of replacing the amino acid residue at position X in the amino acid sequence shown in SEQ ID NO: 2 with a certain amino acid residue" should be read as a "mutation of replacing an amino acid residue corresponding to the amino acid residue at position X in the amino acid sequence shown in SEQ ID NO: 2 with a certain amino acid residue ". That is, for example, in any wild-type TG, "A261C" represents a mutation of replacing an amino acid residue corresponding to A (Ala) residue at position 261 in the amino acid sequence shown as SEQ ID NO: 2 with C (Cys) residue.

Combination of mutations is not particularly limited. Specific examples of combination of mutations include the following combinations:
A261C/A322C, A261C/A322C/S199A, V271C/V326C, M16T/Y34F, and M16T/S 199A.

Particular examples of combination of mutations include A261C/A322C, A261C/A322C/S199A, and V271C/V326C.

More particular examples of combination of mutations include A261C/A322C and A261C/A322C/S199A.

That is, the "specific mutation" may comprise, for example, any of these combinations. The "specific mutation" may consist of, for example, any of these combinations. The "specific mutation" may also consist of, for example, a combination of any of these combinations and other mutation(s) at one or more amino acid residues selected from A261, V271, A322, V326, D46, M16, Y34, W38, and S199.

In the aforementioned notation used for defining combinations, the numbers and the letters at the left and right sides of the numbers represent the same as described above. In the aforementioned notation used for defining combinations, two or more mutations noted together and inserted with "/" represent a double or more multiple mutation. That is, for example, "A261C/A322C" represents a double mutation of A261C and A322C.

The position of amino acid residue referred to in each of the aforementioned mutations is used for convenience to for defining the amino acid residue to be modified, and does not necessarily indicate the absolute position in the wild-type TG. That is, the position of amino acid residue in each of the aforementioned mutations represents the relative position based on the amino acid sequence shown in SEQ ID NO: 2, and the absolute position thereof can shift due to deletion, insertion, addition, or the like of amino acid residue(s). For example, if one amino acid residue is deleted or inserted at a position on the N-terminus side of position X in the amino acid sequence shown as SEQ ID NO: 2, the amino acid residue originally at position X is relocated at position X-1 or X+1, but it is still regarded as the "amino acid residue corresponding to the amino acid residue at position X of the amino acid sequence shown as SEQ ID NO: 2". The amino acid residue before modification referred to in each of the aforementioned mutations is used for convenience to for defining the amino acid residue to be modified, and is not necessarily conserved in the wild-type TG. That is, when the wild-type TG does not have the amino acid sequence shown as SEQ ID NO: 2, there are cases where the amino acid residue before modification referred to in each of the aforementioned mutations is not conserved. That is, each of the aforementioned mutations may also include a mutation of replacing the amino acid residue before modification referred to in each of the aforementioned mutations, wherein the amino acid residue is not conserved, with another amino acid residue (e.g., the amino acid residue after modification referred to in each of the aforementioned mutations). For example, the "mutation at A261" includes not only a mutation of replacing the amino acid residue corresponding to A261, wherein the amino acid residue is conserved (i.e., it is A (Ala) residue), with another amino acid residue, but may also include a mutation of replacing the amino acid residue corresponding to A261, wherein the amino acid residue is not conserved (i.e., it is not A (Ala) residue), with another amino acid residue. The amino acid residue before modification and the amino acid residue after modification are selected so that they are not identical to each other. For any mutant TG, in particular, the amino acid residue before modification referred to in each of the aforementioned mutations may be conserved in the corresponding wild-type TG.

In the amino acid sequence of a certain TG, which amino acid residue is an "amino acid residue corresponding to the amino acid residue at position X in the amino acid sequence shown in SEQ ID NO: 2" can be determined by aligning the amino acid sequence of the certain TG and the amino acid sequence of SEQ ID NO: 2. The alignment can be carried out by, for example, using known gene analysis software. Examples of specific software include DNASIS produced by Hitachi Solutions, GENETYX produced by Genetyx, and so forth (Elizabeth C. Tyler et al., Computers and Biomedical Research, 24 (1) 72-96, 1991; Barton GJ et al., Journal of Molecular Biology, 198 (2), 327-37, 1987).

### <2> Production of mutant TG

The mutant TG can be produced by, for example, allowing a host having the mutant TG gene to express the gene.

The mutant TG can also be produced by, for example, expressing the mutant TG gene in a cell-free protein synthesis system.

Hereafter, production of the mutant TG using the host having the mutant TG gene is explained in detail.

### <2-1> Host

The host having the mutant TG gene can be obtained by introducing the mutant TG gene into an appropriate host. The phrase "introducing a mutant TG gene into a host" also include modifying a TG gene, such as the wild-type TG gene, possessed by the host so as to encode the mutant TG. The phrase "having a mutant TG gene" is also referred to as "having a mutant TG".

The host is not particularly limited, so long as it is able to express a functional mutant TG. Examples of the host include microorganisms, plant cells, insect cells, and animal cells. Particular examples of the host include microorganisms. Examples of the microorganisms include bacteria and yeast. Particular examples of the microorganisms include bacteria.

Examples of the bacteria include bacteria belonging to the family *Enterobacteriaceae,* coryneform bacteria, and *Bacillus* bacteria.

Examples of bacteria belonging to the family *Enterobacteriaceae* include bacteria belonging to the genus *Escherichia, Enterobacter, Pantoea, Klebsiella, Serratia, Erwinia, Photorhabdus, Providencia, Salmonella, Morganella,* or the like. Specifically, bacteria classified into the family *Enterobacteriaceae* according to the taxonomy used in the NCBI (National Center for Biotechnology Information) database (http://www.ncbi.nlm.nih.gov/Taxonomy/Browser/wwwtax.cgi?id=91347) can be used. The *Escherichia* bacteria are not particularly limited, and examples thereof include those classified into the genus *Escherichia* according to the taxonomy known to those skilled in the field of microbiology. Examples of the *Escherichia* bacteria include, for example, those described in the work of Neidhardt et al. (Backmann B.J., 1996, Derivations and Genotypes of some mutant derivatives of Escherichia coli K-12, pp.2460-2488, Table 1, In F.D. Neidhardt (ed.), Escherichia coli and Salmonella Cellular and Molecular Biology/Second Edition, American Society for Microbiology Press, Washington, D.C.). Examples of the *Escherichia* bacteria include, for example, *Escherichia coli.* Specific examples of *Escherichia coli* include, for example, *Escherichia coli* K-12 strains such as W3110 strain (ATCC 27325) and MG1655 strain (ATCC 47076); *Escherichia coli* K5 strain (ATCC 23506); *Escherichia coli* B strains such as BL21 (DE3) strain; and derivative strains thereof. Examples the *Enterobacter* bacterium include, for example, *Enterobacter agglomerans* and *Enterobacter aerogenes.* Examples the *Pantoea* bacteria include, for example, *Pantoea ananatis, Pantoea stewartii, Pantoea agglomerans,* and *Pantoea citrea.* Examples of the *Erwinia* bacteria include *Erwinia amylovora* and *Erwinia carotovora.* Examples of the *Klebsiella* bacteria include *Klebsiella planticola.* The bacteria belonging to the family *Enterobacteriaceae* has been recently reclassified into a plurality of families on the basis of comprehensive comparative genome analysis (Adelou M. et al., Genome-based phylogeny and taxonomy of the 'Enterobacteriales': proposal for Enterobacterales ord. nov. divided into the families Enterobacteriaceae, Erwiniaceae fam. nov., Pectobacteriaceae fam. nov., Yersiniaceae fam. nov., Hafniaceae fam. nov., Morganellaceae fam. nov., and Budviciaceae fam. nov., Int. J. Syst. Evol. Microbiol., 2016, 66:5575-5599). However, in the present invention, bacteria previously the family *Enterobacteriaceae* shall be treated as bacteria belonging to the family *Enterobacteriaceae.*

Examples of the coryneform bacteria include bacteria belonging to the genus *Corynebacterium, Brevibacterium, Microbacterium,* or the like.

Specific examples of such coryneform bacteria include the following species.
*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Corynebacterium ammoniagenes (Corynebacterium stationis)*
*Brevibacterium album*
*Brevibacterium cerinum*
*Microbacterium ammoniaphilum*
Specific examples of the coryneform bacteria include the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens* (*Corynebacterium thermoaminogenes*) AJ12340 (FERM BP-1539)
*Corynebacterium herculis* ATCC 13868
*Brevibacterium divaricatum* (*Corynebacterium glutamicum*) ATCC 14020
*Brevibacterium flavum* (*Corynebacterium glutamicum*) ATCC 13826, ATCC 14067, AJ12418 (FERM BP-2205)
*Brevibacterium immariophilum* ATCC 14068
*Brevibacterium lactofermentum* (*Corynebacterium glutamicum*) ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* (*Corynebacterium stationis*) ATCC 6871, ATCC 6872
*Brevibacterium album* ATCC 15111
*Brevibacterium cerinum* ATCC 15112
*Microbacterium ammoniaphilum* ATCC 15354

The coryneform bacteria include bacteria that had previously been classified into the genus *Brevibacterium,* but are presently united into the genus *Corynebacterium* (Int. J. Syst. Bacteriol., 41, 255 (1991)). Moreover, *Corynebacterium stationis* includes bacteria that had previously been classified as *Corynebacterium ammoniagenes,* but is presently re-classified into *Corynebacterium stationis* on the basis of nucleotide sequence analysis of 16S rRNA etc. (Int. J. Syst. Evol. Microbiol., 60, 874-879 (2010)).

Examples of the *Bacillus* bacteria include, for example, *Bacillus subtilis, Bacillus amyloliquefaciens, Bacillus pumilus, Bacillus licheniformis, Bacillus megaterium, Bacillus brevis, Bacillus polymixa,* and *Bacillus stearothermophilus.* Specific examples of *Bacillus subtilis* include, for example, the *Bacillus subtilis* 168 Marburg strain (ATCC 6051) and PY79 strain (Plasmid, 1984, 12, 1-9). Specific examples of *Bacillus amyloliquefaciens* include, for example, the *Bacillus amyloliquefaciens* T strain (ATCC 23842) and N strain (ATCC 23845).

Examples of the yeast include yeast belonging to the genus *Saccharomyces* such as *Saccharomyces cerevisiae,* the genus *Candida* such as *Candida utilis,* the genus *Pichia* such as *Pichia pastoris,* the genus *Hansenula* such as *Hansenula polymorpha,* and the genus *Schizosaccharomyces* such as *Schizosaccharomyces pombe.*

These strains are available from, for example, the American Type Culture Collection (Address: 12301 Parklawn Drive, Rockville, Maryland 20852, P.O. Box 1549, Manassas, VA 20108, United States of America). That is, registration numbers are given to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

The mutant TG gene can be obtained by, for example, modifying the wild-type TG gene so that the encoded TG has the "specific mutation". The wild-type TG gene to be modified can be obtained by, for example, cloning from an organism having the wild-type TG gene or chemical synthesis. The mutant TG gene can also be obtained without using the wild-type TG gene. For example, the mutant TG gene may be directly obtained by chemical synthesis. The obtained mutant TG gene may be used as it is, or may be further modified before use. For example, a certain embodiment of the mutant TG gene may be modified to obtain another embodiment of the mutant TG gene.

Genes can be modified by using a known method. For example, an objective mutation can be introduced into a target site of DNA by the site-specific mutagenesis method. That is, for example, the coding region of a gene can be modified by the site-specific mutation method so that a specific site of the encoded protein include substitution, deletion, insertion, and/or addition of amino acid residue(s). Examples of the site-specific mutagenesis method include a method of using PCR (Higuchi, R., 61, in PCR Technology, Erlich, H.A. Eds., Stockton Press, 1989; Carter P., Meth., in Enzymol., 154, 382, 1987), and a method of using a phage (Kramer, W. and Frits, H.J., Meth. in Enzymol., 154, 350, 1987; Kunkel, T.A. et al., Meth. in Enzymol., 154, 367, 1987).

Methods for introducing the mutant TG gene into the host are not particularly limited. It is sufficient that the mutant TG gene is harbored by the host so that the gene is able to be expressed. That is, it is sufficient that the mutant TG gene is harbored by the host so that the gene is able to be expressed under control of a promoter that functions in the host. In the host, the mutant TG gene may be present on a vector autonomously replicable outside a chromosome, such as a plasmid, or may be incorporated into a chromosome. The host may have one copy or two or more copies of the mutant TG gene. The host may have one kind of mutant TG gene, or two or more kinds of mutant TG genes.

The promoter for expressing the mutant TG gene is not particularly limited, so long as it functions in the host. The term "promoter that functions in a host" refers to a promoter that shows a promoter activity in the host. The promoter may be a promoter derived from the host, or a heterogenous promoter. The promoter may be the native promoter of the TG gene, or a promoter of another gene. The promoter may be a stronger promoter than the native promoter of the TG gene. Examples of stronger promoters that function in bacteria of the family *Enterobacteriaceae* such as *Escherichia coli* can include, for example, T7 promoter, *trp* promoter, *trc* promoter, *lac* promoter, *tac* promoter, *tet* promoter, *araBAD* promoter, *rpoH* promoter, *msrA* promoter, Pm1 promoter (derived from the genus *Bifidobacterium*)*,* PR promoter, and PL promoter. Examples of stronger promoters that function in coryneform bacteria can include, for example, the artificially modified P54-6 promoter (Appl. Microbiol. Biotechnol., 53, 674-679 (2000)), *pta, aceA, aceB, adh,* and *amyE* promoters inducible in coryneform bacteria with acetic acid, ethanol, pyruvic acid, or the like, and *cspB, SOD,* and *tuf* (EF-Tu) promoters, which are potent promoters capable of providing a large expression amount in coryneform bacteria (Journal of Biotechnology, 104 (2003) 311-323; Appl. Environ. Microbiol., 2005 Dec; 71 (12):8587-96), as well as P2 promoter (WO2018/079684), P3 promoter (WO2018/079684), *lac* promoter, *tac* promoter, *trc* promoter, and F1 promoter (WO2018/179834). Furthermore, as the stronger promoter, a highly-active type of an inherent promoter may also be obtained by using various reporter genes and used. For example, by making the -35 and -10 regions in a promoter region closer to the consensus sequence, the activity of the promoter can be enhanced (WO00/18935). Examples of highly active-type promoter can include various tac-like promoters (Katashkina JI et al., Russian Federation Patent Application No. 2006134574) and pnlp8 promoter (WO2010/027045). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

Furthermore, a terminator for termination of gene transcription may be located downstream of the mutant TG gene. The terminator is not particularly limited so long as it functions in the host. The terminator may be a terminator derived from the host, or a heterogenous terminator. The terminator may be the native terminator of the TG gene, or a terminator of another gene. Specific examples of the terminator can include, for example, T7 terminator, T4 terminator, fd phage terminator, *tet* terminator, and *trpA* terminator.

The mutant TG gene may be introduced into the host by, for example, using a vector containing the gene. The vector containing the mutant TG gene is also referred to as an expression vector or recombinant vector of the mutant TG gene. The expression vector of the mutant TG gene can be constructed by, for example, ligating a DNA fragment containing the mutant TG gene with a vector that functions in the host. By transforming the host with the expression vector of the mutant TG gene, the host introduced with the vector can be obtained, that is, the gene can be introduced into the host. As the vector, a vector autonomously replicable in the cell of the host can be used. It is preferred that the vector is a multi-copy vector. Furthermore, it is preferred that the vector has a marker such as an antibiotic resistance gene for selection of a transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vector autonomously replicable in *Enterobacteriaceae* bacteria such as *Escherichia coli* can include, for example, pUC19, pUC18, pHSG299, pHSG399, pHSG398, pBR322, pSTV29 (all of these are available from Takara), pACYC184, pMW219 (NIPPON GENE), pTrc99A (Pharmacia), pPROK series vectors (Clontech), pKK233-2 (Clontech), pET series vectors (Novagen), pQE series vectors (QIAGEN), pCold TF DNA (Takara Bio), pACYC series vectors, and the broad host spectrum vector RSF1010. Specific examples of vector autonomously replicable in coryneform bacteria can include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 described in Japanese Patent Laid-open (Kokai) No. 3-210184; plasmids pCRY21, pCRY2KE, pCRY2KX, pCRY31, pCRY3KE, and pCRY3KX described in Japanese Patent Laid-open (Kokai) No. 2-72876 and U.S. Patent No. 5,185,262; plasmids pCRY2 and pCRY3 described in Japanese Patent Laid-open (Kokai) No. 1-191686; pAJ655, pAJ611, and pAJ1844 described in Japanese Patent Laid-open (Kokai) No. 58-192900; pCG1 described in Japanese Patent Laid-open (Kokai) No. 57-134500; pCG2 described in Japanese Patent Laid-open (Kokai) No. 58-35197; pCG4 and pCG11 described in Japanese Patent Laid-open (Kokai) No. 57-183799; pPK4 described in U.S. Patent No. 6,090,597; pVK4 described in Japanese Patent Laid-open (Kokai) No. 9-322774; pVK7 described in Japanese Patent Laid-open (Kokai) No. 10-215883; pVK9 described in WO2007/046389; pVS7 described in WO2013/069634; and pVC7 described in Japanese Patent Laid-open (Kokai) No. 9-070291. Specific examples of vector autonomously replicable in coryneform bacteria can also include, for example, variants of pVC7 such as pVC7H2 (WO2018/179834). Upon construction of the expression vector, for example, the mutant TG gene containing a native promoter region may be integrated into a vector as it is, a coding region of the mutant TG gene may be ligated downstream of such a promoter as mentioned above and then may be integrated into a vector, or a coding region of the mutant TG gene may be integrated downstream of a promoter inherently present on a vector.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

The mutant TG gene can also be introduced into the chromosome of the host. The gene can be introduced into the chromosome by, for example, using homologous recombination (Miller, J.H., Experiments in Molecular Genetics, 1972, Cold Spring Harbor Laboratory). Examples of the gene transfer method utilizing homologous recombination can include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Only one copy, or two or more copies of a gene may be introduced. For example, by carrying out homologous recombination using a sequence which is present in multiple copies on the chromosome as a target, multiple copies of the gene can be introduced into the chromosome. Examples of such a sequence which is present in multiple copies on the chromosome can include repetitive DNAs, and inverted repeats located at the both ends of a transposon. Alternatively, homologous recombination may be carried out by using an appropriate sequence on the chromosome such as a gene unnecessary for carrying out the present invention as a target. Furthermore, the gene can also be randomly introduced into the chromosome by using a transposon or Mini-Mu (Japanese Patent Laid-open (Kokai) No. 2-109985, U.S. Patent No. 5,882,888, EP 805867 B1). Upon introduction of the gene into the chromosome, for example, the mutant TG gene containing a native promoter region may be integrated into the chromosome as it is, a coding region of the mutant TG gene may be ligated downstream of such a promoter as mentioned above and then may be integrated into the chromosome, or a coding region of the mutant TG gene may be integrated downstream of a promoter inherently present on the chromosome.

Introduction of the gene into the chromosome can be confirmed by, for example, Southern hybridization using a probe having a nucleotide sequence complementary to the whole or a part of the gene, or PCR using primers prepared on the basis of the sequence of the gene.

The transformation method is not particularly limited, and conventionally known methods can be used. Examples of the transformation method include, for example, a method of treating recipient cells with calcium chloride so as to increase the permeability thereof for DNA, which has been reported for the *Escherichia coli* K-12 strain (Mandel, M. and Higa, A., J. Mol. Biol., 1970, 53, 159-162), a method of preparing competent cells from cells which are in the growth phase, followed by transformation with DNA, which has been reported for *Bacillus subtilis* (Duncan, C.H., Wilson, G.A. and Young, F.E., Gene, 1977, 1:153-167), and so forth. Furthermore, as the transformation method, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis,* actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, as the transformation method, the electric pulse method reported for coryneform bacteria (Japanese Patent Laid-open (Kokai) No. 2-207791) can also be used.

In addition, when the host already has a TG gene, such as the wild-type TG gene, on the chromosome thereof or the like, the TG gene can also be modified to encode the mutant TG, so that the host has the mutant TG gene. The term "introduction of a mutant TG gene" may also include modifying a TG gene possessed by the host to the mutant TG gene. Modification of a TG gene present on the chromosome or the like can be carried out by, for example, natural mutation, mutagenesis treatment, or genetic engineering.

The host may or may not have the wild-type TG gene. In particular, it is acceptable that the host does not have the wild-type TG gene.

The host may have any characteristics, so long as the mutant TG can be produced.

### <2-2> Cultivation of host

By culturing the host having the mutant TG gene, the mutant TG can be expressed.

The culture medium to be used is not particularly limited, so long as the host can proliferate in it and express the functional mutant TG. As the culture medium, for example, a usual culture medium used for culture of microorganisms such as bacteria and yeast can be used. The culture medium may contain culture medium components such as carbon source, nitrogen source, phosphate source, and sulfur source, as well as other various organic components and inorganic components as required. The types and concentrations of the culture medium components can be appropriately set according to various conditions such as the type of the host.

Specific examples of the carbon source include, for example, saccharides such as glucose, fructose, sucrose, lactose, galactose, xylose, arabinose, blackstrap molasses, hydrolysates of starches, and hydrolysates of biomass; organic acids such as acetic acid, citric acid, succinic acid, and gluconic acid; alcohols such as ethanol, glycerol, and crude glycerol; and fatty acids. As the carbon source, plant-derived materials can be preferably used. Examples of the plant include, for example, corn, rice, wheat, soybean, sugarcane, beet, and cotton. Examples of the plant-derived materials include, for example, organs such as root, stem, trunk, branch, leaf, flower, and seed, plant bodies including them, and decomposition products of these plant organs. The forms of the plant-derived materials at the time of use thereof are not particularly limited, and they can be used in any form such as unprocessed product, juice, ground product, and purified product. Pentoses such as xylose, hexoses such as glucose, or mixtures of them can be obtained from, for example, plant biomass, and used. Specifically, these saccharides can be obtained by subjecting a plant biomass to such a treatment as steam treatment, hydrolysis with concentrated acid, hydrolysis with diluted acid, hydrolysis with an enzyme such as cellulase, and alkaline treatment. Since hemicellulose is generally more easily hydrolyzed compared with cellulose, hemicellulose in a plant biomass may be hydrolyzed beforehand to liberate pentoses, and then cellulose may be hydrolyzed to generate hexoses. Further, xylose may be supplied by conversion from hexoses by, for example, imparting a pathway for converting hexose such as glucose to xylose to the host. As the carbon source, one kind of carbon source may be used, or two or more kinds of carbon sources may be used in combination.

The concentration of the carbon source in the culture medium is not particularly limited, so long as the host can proliferate in it and express the functional mutant TG. The concentration of the carbon source in the culture medium may be as high as possible within such a range that production of the mutant TG is not inhibited. Initial concentration of the carbon source in the culture medium may be, for example, usually 5 to 30 w/v%, preferably 10 to 20 w/v%. Further, the carbon source may be additionally supplied to the culture medium as required. For example, the carbon source may be additionally supplied to the culture medium in proportion to decrease or depletion of the carbon source accompanying progress of the cultivation. While the carbon source may be temporarily depleted so long as the mutant TG can be eventually produced, it may be preferable to carry out the culture so that the carbon source is not depleted or the carbon source does not continue to be depleted.

Specific examples of the nitrogen source include, for example, ammonium salts such as ammonium sulfate, ammonium chloride, and ammonium phosphate, organic nitrogen sources such as peptone, yeast extract, meat extract, and soybean protein decomposition products, ammonia, and urea. Ammonia gas and aqueous ammonia used for pH adjustment may also be used as a nitrogen source. As the nitrogen source, one kind of nitrogen source may be used, or two or more kinds of nitrogen sources may be used in combination.

Specific examples of the phosphate source include, for example, phosphate salts such as potassium dihydrogenphosphate and dipotassium hydrogenphosphate, and phosphoric acid polymers such as pyrophosphoric acid. As the phosphate source, one kind of phosphate source may be used, or two or more kinds of phosphate sources may be used in combination.

Specific examples of the sulfur source include, for example, inorganic sulfur compounds such as sulfates, thiosulfates, and sulfites, and sulfur-containing amino acids such as cysteine, cystine, and glutathione. As the sulfur source, one kind of sulfur source may be used, or two or more kinds of sulfur sources may be used in combination.

Specific examples of other various organic and inorganic components include, for example, inorganic salts such as sodium chloride and potassium chloride; trace metals such as iron, manganese, magnesium, and calcium; vitamins such as vitamin B 1, vitamin B2, vitamin B6, nicotinic acid, nicotinamide, and vitamin B12; amino acids; nucleic acids; and organic components containing these such as peptone, casamino acid, yeast extract, and soybean protein decomposition product. As the other various organic and inorganic components, one kind of component may be used, or two or more kinds of components may be used in combination.

Furthermore, when an auxotrophic mutant strain that requires a nutrient such as amino acids for growth thereof is used, it is preferable to supplement such a required nutrient to the culture medium.

Culture conditions are not particularly limited, so long as the host can proliferate in it and express the functional mutant TG. The culture can be carried out with, for example, usual conditions used for culture of microorganisms such as bacteria and yeast. The culture conditions may be appropriately set according to various conditions such as the type of the host. In addition, expression of the mutant TG gene may be induced, as required.

The culture can be carried out by using a liquid culture medium. At the time of the culture, for example, the host cultured on a solid culture medium such as agar medium may be directly inoculated into a liquid culture medium, or the host cultured in a liquid culture medium as seed culture may be inoculated into a liquid culture medium for main culture. That is, the culture may be carried out separately as seed culture and main culture. In such a case, the culture conditions of the seed culture and the main culture may or may not be the same. It is sufficient that the mutant TG is expressed at least during the main culture. The amount of the host contained in the culture medium at the start of the culture is not particularly limited. For example, a seed culture broth showing an OD660 of 4 to 100 may be added to a culture medium for main culture in an amount of 0.1 to 100 mass %, preferably 1 to 50 mass %, at the start of the culture.

The culture can be carried out as batch culture, fed-batch culture, continuous culture, or a combination of these. The culture medium used at the start of the culture is also referred to as "starting medium". The culture medium supplied to the culture system (e.g., fermentation tank) in the fed-batch culture or the continuous culture is also referred to as "feed medium". To supply a feed medium to the culture system in the fed-batch culture or the continuous culture is also referred to as "feed". Furthermore, when the culture is carried out separately as seed culture and main culture, the culture schemes of the seed culture and the main culture may or may not be the same. For example, both the seed culture and the main culture may be carried out as batch culture. Alternatively, for example, the seed culture may be carried out as batch culture, and the main culture may be carried out as fed-batch culture or continuous culture.

In the present invention, the various components such as the carbon source may be contained in the starting medium, feed medium, or both. That is, the various components such as the carbon source may be additionally supplied to the culture medium independently or in any combination during the culture. These components each may be supplied once or a plurality of times, or may be continuously supplied. The types of the components contained in the starting medium may or may not be the same as those of the components contained in the feed medium. Furthermore, the concentrations of the components contained in the starting medium may or may not be the same as the concentrations of the components contained in the feed medium. Furthermore, two or more kinds of feed media containing components of different types and/or different concentrations may be used. For example, when feeding is intermittently carried out two or more times, the types and/or concentrations of components contained in the feed medium may or may not be the same for each feeding.

The culture can be carried out, for example, under aerobic conditions. The term "aerobic conditions" may mean that the dissolved oxygen concentration in the culture medium is 0.33 ppm or higher, or preferably 1.5 ppm or higher. The oxygen concentration can be controlled to be, for example, 1 to 50%, preferably about 5%, of the saturated oxygen concentration. The culture can be carried out, for example, with aeration or shaking. The pH of the culture medium may be, for example, pH 3 to 10, preferably pH 4.0 to 9.5. The pH of the culture medium can be adjusted during the culture as required. The pH of the culture medium can be adjusted by using various alkaline and acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 45°C, preferably 25 to 37°C. The culture time may be, for example, 10 to 120 hours. The culture may be continued, for example, until the carbon source contained in the culture medium is consumed, or until the activity of the host is lost.

By culturing the host as described above, a culture broth containing the mutant TG is obtained. The mutant TG may be accumulated in, for example, microbial cells of the host. The term "microbial cell" may be appropriately read as "cell" depending on the type of the host. Furthermore, depending on the type of the host and/or design of the mutant TG gene, for example, the mutant TG may be accumulated in the periplasm, or may be secreted outside the cells.

The mutant TG may be used as it is contained in the culture broth (specifically, the culture medium or cells), or after being purified from the culture broth (specifically, the culture medium or cells). The purification can be carried out to a desired extent. That is, examples of the mutant TG include a purified mutant TG and a fraction containing the mutant TG. In other words, the mutant TG may be used in the form of a purified enzyme, may be used in the form of such a fraction (i.e., in the form contained in such a fraction), or may be used in the form of a combination thereof. Such a fraction is not particularly limited, so long as it contains the mutant TG in such a manner that the mutant TG can act on the substrate thereof. Examples of such a fraction include a culture broth of the host having the mutant TG gene (i.e., the host having the mutant TG), cells collected from the culture broth, a supernatant collected from the culture broth, a processed product thereof (e.g., a processed product of cells such as cell disrupt, cell lysate, cell extract, and immobilized cells), and combinations thereof. The term "purified mutant TG" may include a roughly-purified product. Any of these fractions may be independently used, or may be used in combination with a purified mutant TG. The mutant TG may also be used, for example, in the form of not contained in the cells. The mutant TG may also be used, for example, in the form of the composition of the present invention described below.

When the mutant TG is accumulated in the culture medium, for example, a culture supernatant can be obtained by centrifugation or the like, and then the mutant TG can be purified from the culture supernatant. Also, when the mutant TG is accumulated in the cells of the host, for example, the cells can be subject to a treatment such as disruption, lysis, and extraction, and then the mutant TG can be purified from the treated product. The mutant TG can be purified by, for example, known methods used for purification of proteins. Examples of such methods include ammonium sulfate fractionation, ion exchange chromatography, hydrophobic chromatography, affinity chromatography, gel filtration chromatography, and isoelectric precipitation. These methods can be independently used, or can be used in combination as required.

The use purpose of the mutant TG is not particularly limited.

The mutant TG may be used for, for example, modification of a food. The mutant TG may also be used for, for example, production of a food. The food to be produced by using the mutant TG may be a modified food. That is, specifically, the mutant TG may be used for, for example, production of a modified food. In other words, a modified food may be obtained by modification of a food.

Examples of the food include foods whose production is accompanied by a decrease in pH. Examples of the food also include foods produced by fermentation. The fermentation can be carried out by, for example, using a microorganism such as lactic acid bacteria and yeasts. The pH may be decreased, for example, due to the fermentation. That is, the foods whose production is accompanied by a decrease in pH can be examples of the foods produced by fermentation. Also, the foods produced by fermentation can be examples of the foods whose production is accompanied by a decrease in pH. Specific examples of the food include yogurt and cheese. Particular examples of the food include yogurt. Yogurt and cheese each can be an example of the foods whose production is accompanied by a decrease in pH and/or the foods produced by fermentation

Furthermore, a food produced by using the mutant TG may be used as a raw material, to produce another food. For convenience of explanation, a food produced by using the mutant TG is also referred to as "intermediate product", and another food produced by using an intermediate product as a raw material is also referred to as "final product". That is, examples of the food also include such a final product. Examples of the final product include foods containing such a food as exemplified above (e.g., yogurt or cheese). Examples of the foods containing yogurt or cheese include ice cream containing yogurt or cheese. Production of a final product by using an intermediate product as a raw material may also result in modification of the final product. The type of modification of the final product may or may not be identical to the type of modification of the intermediate product. The phrase "using a mutant TG for modification or production of a food" may also include cases where the mutant TG is indirectly used for modification or production of a final product via production of the final product by using an intermediate product as a raw material.

Examples of the modification of foods include improvement of physical properties. Examples of the improvement of physical properties in the case of yogurt or cheese include an increase in breaking strength, prevention of water separation, and impartation of a smooth texture. Particular examples of the improvement of physical properties in the case of yogurt or cheese include an increase in breaking strength. That is, for example, by using the mutant TG, an effect of improving physical properties may be obtained in a food such as yogurt or cheese, as compared to cases of not using the mutant TG. Specifically, for example, by producing a food such as yogurt or cheese using the mutant TG, a food having an improved physical property, such as yogurt or cheese whose breaking strength is increased, whose water separation is prevented, and/or to which a smooth texture is imparted, may be obtained, as compared to cases of producing the food such as yogurt or cheese without using the mutant TG.

### <3> Composition of the present invention

The composition of the present invention is a composition containing the mutant TG.

The composition of the present invention may be, for example, a composition to be used for such a use purpose of the mutant TG as exemplified above. That is, the composition of the present invention may be, for example, a composition for modifying a food such as yogurt or cheese. The composition of the present invention may also be, for example, a composition for producing a food such as yogurt or cheese. The composition of the present invention may also be, specifically, for example, a composition for producing a modified food such as modified yogurt or modified cheese.

The composition of the present invention may consist of the mutant TG, or may contain ingredient(s) other than the mutant TG.

The ingredients other than the mutant TG are not particularly limited, so long as the function of the mutant TG is not spoiled. As the ingredients other than the mutant TG, those acceptable depending on the use purpose of the composition of the present invention can be used. Examples of the ingredients other than the mutant TG include ingredients to be blended in foods or pharmaceuticals.

As the composition of the present invention, for example, the mutant TG in such a mode as exemplified above may be used as it is, or may be used after made into a formulation as required. For preparing such a formulation, additive(s) acceptable depending on the use purpose of the composition of the present invention can be used as required. Examples of the additives include excipients, binders, disintegrating agents, lubricants, stabilizers, corrigents, perfumes, diluents, surfactants, and solvents. The additive(s) can be appropriately selected, for example, according to various conditions such as the form of the composition of the present invention.

The form of the composition of the present invention is not particularly limited. The composition of the present invention may be provided, for example, in any form such as the form of powder, flake, tablet, paste, or liquid.

### <4> Method of the present invention

The method of the present invention is a method of using the mutant TG.

In the method of the present invention, the mutant TG may be used for, for example, such a use purpose of the mutant TG as exemplified above. That is, the method of the present invention may be, for example, a method for modifying a food such as yogurt or cheese. The method of the present invention may also be, for example, a method for producing a food such as yogurt or cheese. The method of the present invention may also be, specifically, for example, a method for producing a modified food such as modified yogurt or modified cheese.

In modification or production of a food, the mutant TG may be used for treating a food raw material. The term "food raw material" may refer to a material to be used as a raw material for producing a food. The food raw material can be appropriately selected according to various conditions such as the type of food. Examples of the food raw material include a milk raw material. For example, in cases where the food is yogurt or cheese, the food raw material may be a milk raw material. Hereafter, the method of the present invention will be explained mainly in reference to cases where the food is yogurt. Such explanation can be similarly applied to other foods. In such a case, the "milk raw material" in modification or production of yogurt can be read as the "food raw material" in modification or production of other foods.

In modification or production of yogurt, the mutant TG may be used for treating the milk raw material. That is, examples of use of the mutant TG include treating the milk raw material with the mutant TG. That is, the method of the present invention may be, for example, a method for modifying yogurt, the method comprising a step of treating the milk raw material with the mutant TG. The method of the present invention may also be, for example, a method for producing yogurt, the method comprising a step of treating the milk raw material with the mutant TG. The method of the present invention may also be, specifically, for example, a method for producing modified yogurt, the method comprising a step of treating the milk raw material with the mutant TG. The phrase "treating a milk raw material with a mutant TG" is also referred to as "making a mutant TG act on a milk raw material". The step of "treating a milk raw material with a mutant TG" is also referred to as "treatment step". That is, the method of the present invention may comprise the treatment step.

The mutant TG may be used for treating the milk raw material in any form that allows the mutant TG to act on the milk raw material. The mutant TG may be used for treating the milk raw material, for example, in such a form as exemplified above. The mutant TG may be used for treating the milk raw material, specifically, for example, in the form of the composition of the present invention. That is, the phrase "treating a milk raw material with a mutant TG" also includes cases of treating the milk raw material with the composition of the present invention.

Modification or production of yogurt may be carried out, for example, in the same manner as modification or production of usual yogurt, except that the mutant TG is used. Modification or production of yogurt may be carried out, for example, in particular, in the same manner as modification or production of yogurt using TG, which is described in WO2018/079687, WO2018/079686, WO2018/079685, WO2018/079684, WO2018/079683, WO2017/073701, WO2018/079705, US2018-0334693A, US2019-0161776A, or the like, except that the mutant TG is used as TG.

Modification or production of yogurt may be carried out, specifically, for example, by using the same milk raw material and the same production conditions as those for usual yogurt, except that the mutant TG is used. Also, the milk raw material and the production conditions each may also be modified as required and then used for modification or production of yogurt. The method of the present invention may comprise a step of producing yogurt from the milk raw material. This step is also referred to as "production step of yogurt". The treatment step may also be a step of treating the milk raw material with the mutant TG to produce yogurt.

The term "milk raw material" may refer to a raw material containing a milk protein. The milk raw material is not particularly limited, so long as yogurt can be produced using the milk raw material by the method of the present invention. Examples of the milk protein include casein and whey proteins. Examples of the whey proteins include alpha-lactalbumin and beta-lactoglobulin. The milk raw material may contain one or more of these milk proteins, or may contain all of these milk proteins. The contained amount (concentration) of the milk protein in the milk raw material can be measured by, for example, measuring the total nitrogen content by the Kjeldahl method and multiplying the total nitrogen content by the nitrogen-to-protein conversion factor. In the case of the milk protein, the nitrogen-to-protein conversion factor may be 6.38. Examples of milk include cow's milk, goat's milk, sheep's milk, buffalo's milk, reindeer's milk, donkey's milk, and camel's milk. Particular examples of milk include cow's milk. As the milk raw material, for example, milk can be used as it is, or after appropriate modification. That is, the milk raw material may or may not be raw milk (fresh milk). The milk raw material, for example, may or may not have been subject to a treatment such as heating, homogenization, and drying. The milk raw material, for example, may or may not have been ingredient-adjusted. Specific examples of the milk raw material include whole milk, skimmed milk, partially skimmed milk, buttermilk, processed products thereof, and ingredient-adjusted products thereof. Examples of the ingredient-adjusted products include calcium-fortified milk. As the milk raw material, one raw material may be used, or two or more raw materials may be used in combination.

Yogurt can be produced by fermentation. The fermentation can be carried out by, for example, using a starter. Fermentation can be carried out by, specifically, for example, making a starter coexist with the milk raw material. The pH of the milk raw material at the start of fermentation, for example, may be 6.0 or higher, may be 7.4 or lower, 7.2 or lower, or 7.0 or lower, or may be within a range defined as a combination thereof. The pH of the milk raw material at the start of fermentation may be, specifically, for example, 6.0 to 7.0.

The term "starter" refers to a microorganism to be used for the fermentation. The starter is not particularly limited, so long as yogurt can be produced using the starter by the method of the present invention. Examples of the starter include lactic acid bacteria and yeasts. Particular examples of the starter include lactic acid bacteria.

Examples of the lactic acid bacteria include *Lactobacillus* bacteria, *Lactococcus* bacteria, *Streptococcus* bacteria, and *Bifidobacterium* bacteria.

Examples of the *Lactobacillus* bacteria include *Lactobacillus delbrueckii, Lactobacillus casei, Lactobacillus paracasei, Lactobacillus gasseri,* and *Lactobacillus acidophilus. Lactobacillus delbrueckii* includes strains classified to any subspecies of *Lactobacillus delbrueckii,* such as *Lactobacillus delbrueckii* subsp. bulgaricus, *Lactobacillus delbrueckii* subsp. delbrueckii, and *Lactobacillus delbrueckii* subsp. lactis.

Examples of the *Lactococcus* bacteria include *Lactococcus lactis. Lactococcus lactis* includes strains classified to any subspecies of *Lactococcus lactis,* such as *Lactococcus lactis* subsp. lactis, *Lactococcus lactis* subsp. cremoris, and *Lactococcus lactis* subsp. hordniae.

Examples of the *Streptococcus* bacteria include *Streptococcus thermophilus.*

Examples of the *Bifidobacterium* bacteria include *Bifidobacterium longum, Bifidobacterium breve, Bifidobacterium bifidum, Bifidobacterium adolescentis, Bifidobacterium angulatum, Bifidobacterium dentium, Bifidobacterium pseudocatenulatum, Bifidobacterium animalis, Bifidobacterium pseudolongum,* and *Bifidobacterium thermophilum.*

As the starter, one microorganism may be used, or two or more microorganisms may be used in combination.

As the starter, for example, a commercially available starter may be used. As the starter, for example, a microorganism obtained from depository or distribution institute may also be used.

The fermentation may be carried out, for example, until the pH of the fermentation product decreases to a desired extent. The pH of the fermentation product at the completion of fermentation, for example, may be 5.0 or lower, 4.8 or lower, 4.6 or lower, 4.4 or lower, or 4.2 or lower, may be 3.8 or higher, 4.0 or higher, 4.2 or higher, or 4.4 or higher, or may be within a range defined as a non-contradictory combination thereof. The pH of the fermentation product at the completion of fermentation may be, specifically, for example, 4.0 to 4.6. The term "fermentation product" may refer to the milk raw material after the start of fermentation, and may also include yogurt eventually obtained. The term "fermentation product at the completion of fermentation" may refer to yogurt eventually obtained. The fermentation period, for example, may be 1 hour or longer, 2 hours or longer, 3 hours or longer, 4 hours or longer, 5 hours or longer, 7 hours or longer, 10 hours or longer, 15 hours or longer, or 20 hours or longer, may be 30 hours or shorter, 25 hours or shorter, 20 hours or shorter, 15 hours or shorter, 10 hours or shorter, or 7 hours or shorter, or may be within a range defined as a non-contradictory combination thereof. The fermentation temperature, for example, may be 25°C or higher, 30°C or higher, 35°C or higher, or 40°C or higher, may be 45°C or lower, 40°C or lower, or 35°C or lower, or may be within a range defined as a non-contradictory combination thereof.

By carrying out the fermentation as described above, yogurt is prepared.

The mutant TG may be made act on the milk raw material at any stage of the production step of yogurt, so long as a desired effect, such as an effect of modifying yogurt, is obtained. The mutant TG can be made act on the milk raw material by making the mutant TG coexist with the milk raw material as it is or after prepared as a desired form such as the form of liquid. For example, the mutant TG may be added to the milk raw material, or a treatment solution containing the mutant TG may be mixed with the milk raw material. Such operations for making the mutant TG coexist with the milk raw material are collectively referred to as "addition" of the mutant TG. The mutant TG may be added, for example, before the start of fermentation, at the start of fermentation, or after the start of fermentation. The mutant TG may be added, specifically, for example, before the addition of the starter, at the addition of the starter, or after the addition of the starter. The mutant TG may be added, for example, before the completion of fermentation, at the completion of fermentation, or after the completion of fermentation. In particular, the mutant TG may be added before the completion of fermentation. The mutant TG may be added, specifically, before the pH of the fermentation product decreases to a desired extent. The pH of the fermentation product at the addition of the mutant TG, for example, may be 5.0 or higher, 5.2 or higher, 5.4 or higher, 5.6 or higher, 5.8 or higher, or 6.0 or higher, may be 7.4 or lower, 7.2 or lower, 7.0 or lower, 6.8 or lower, 6.6 or lower, or 6.4 or lower, or may be within a range defined as a non-contradictory combination thereof. The pH of the fermentation product at the addition of the mutant TG may be, specifically, for example, 6.0 to 7.0. Depending on various conditions such as the timing of the addition of the mutant TG and the length of a period during which the activity of the mutant TG remains, the fermentation of the milk raw material proceeds during the treatment with the mutant TG. Hence, the "milk raw material" to be treated with the mutant TG is not limited to the milk raw material before the start of fermentation, but may also include the fermentation product.

Conditions for carrying out the treatment step are not particularly limited, so long as a desired effect, such as an effect of modifying yogurt, is obtained. The conditions for carrying out the treatment step can be appropriately set, for example, according to various conditions such as the characteristics and the addition amount of the mutant TG. The treatment step may be carried out, for example, in conjunction with the fermentation. That is, the production step of yogurt may serve as the treatment step. However, depending on various conditions such as the timing of the addition of the mutant TG and the length of a period during which the activity of the mutant TG remains, a part or the whole of the treatment step may be carried out before the fermentation, or a part or the whole of the treatment step may be carried out after the fermentation. For example, the aforementioned descriptions concerning the fermentation temperature and the fermentation period can also be applied similarly to the temperature and the period of the treatment step.

The mutant TG may be deactivated, for example, during production of yogurt. The mutant TG may be deactivated, specifically, for example, during the fermentation. The mutant TG may be deactivated, more specifically, for example, due to decrease in the pH of the fermentation product. The remaining activity of the mutant TG in the fermentation product at the completion of fermentation may be, for example, 20% or less, 10% or less, 5% or less, 3% or less, 2% or less, 1% or less, or 0%. In other words, the fermentation may be carried out until the remaining activity of the mutant TG decreases to the aforementioned range. The phrase "remaining activity of a mutant TG in a fermentation product at the completion of fermentation" refers to the ratio of the TG activity of the mutant TG in the fermentation product at the completion of fermentation to the TG activity of the mutant TG at the addition thereof. According to the present invention, in particular, the mutant TG may be deactivated without carrying out a heat treatment. Hence, for example, it is acceptable that the method of the present invention does not comprise a treatment for deactivating the mutant TG by heating. If the mutant TG is sufficiently deactivated, for example, unnecessary modification of the physical properties of yogurt by the mutant TG after the completion of fermentation (e.g., during storage or distribution of the final product, yogurt) can possibly be prevented. When a food is produced with addition of an enzyme, the enzyme that remains in the final product without being deactivated is required to be labeled as a food additive. However, if the mutant TG is sufficiently deactivated, for example, it can possibly no longer be necessary to label an "enzyme" as a food additive in the final product.

In the method of the present invention, ingredient(s) other than the ingredients exemplified above (e.g., the milk raw material, the starter, and the mutant TG) may also be used, so long as a desired effect, such as an effect of modifying yogurt, is obtained. Examples of such ingredients include raw materials that can be used for production of yogurt and are other than the ingredients exemplified above. Such ingredients each may be preliminarily mixed with the milk raw material, or may be appropriately added to the milk raw material during production of yogurt.

The addition amounts and addition ratios of the ingredients in the method of the present invention are not particularly limited, so long as a desired effect, such as an effect of modifying yogurt, is obtained. The addition amounts and addition ratios of the ingredients in the method of the present invention can be appropriately set according to various conditions such as the types of the ingredients.

The addition amount of the mutant TG, for example, may be 0.00033 U or more, 0.001 U or more, 0.0033 U or more, 0.01 U or more, 0.033 U or more, 0.1 U or more, 0.33 U or more, or 1 U or more, may be 100 U or less, 33 U or less, 10 U or less, 3.3 U or less, or 1 U or less, or may be within a range defined as a non-contradictory combination thereof, per 1 g of the milk protein. The addition amount of the mutant TG may be, specifically, for example, 0.00033 U to 33 U or 0.0033 U to 3.3 U per 1 g of the milk protein.

### Examples

Hereinafter, the present invention will be more specifically explained with reference to non-limiting examples.

### Example 1: Construction and analysis of mutant transglutaminases (mutant TGs)

In this Example, mutant TGs were constructed and analyzed.

### <1> Experimental methods

Quantification of TG and measurement of the TG activity in this Example were carried out according to the following procedure, unless otherwise stated.

### <1-1> Quantification of TG

Quantification of TG was carried out by HPLC analysis using BSA as a standard. Analysis conditions are shown below.

Mobile phase A: 0.1% trifluoroacetic acid (TFA)
Mobile phase B: 0.1% TFA, 80% acetonitrile
Flow rate: 1.0 ml/min
Column temperature: 40°C
Detection: UV 280 nm
Column: Proteonavi, 4.6 x 150 mm, 5 µm (OSAKA SODA CO., LTD.)
Gradient: 0 min (B: 30%), 0-20 min (B: 30-50%), 20-25 min (B: 50-100%), 25-26 min
(B: 100%), 26-27 min (B: 100-30%), 27-30 min (B: 30%)

### <1-2> Measurement of TG activity

Measurement of the TG activity was carried out by the hydroxamate method. Solution A (50 mM MES, 100 mM NH₂OH, 10 mM glutathione (reduced form), 30 mM CBZ-Gln-Gly (Z-QG), adjusted to pH 6.0 with NaOH) (500 µL) was added in a 1.5 mL tube, and heated at 37°C for 5 min. After 50 µL of an enzyme solution was added and the reaction was carried out at 37°C for 10 min, 500 µL of solution B (1N HCl, 4% TCA, 1.67% FeCl₃-6H₂O) was added to stop the reaction. A reaction stop solution (200 µL) was added to a 96-well plate, and the absorbance at 525 nm was measured using a plate reader. As a control, the absorbance was measured for a resulting mixture similarly obtained by a reaction using 20 mM MES pH 6.0, and the absorbance difference from the sample solution was determined. Separately, a calibration curve was prepared using L-glutamic acid-γ-monohydroxamate instead of the enzyme solution, the amount of hydroxamic acid produced was determined from the absorbance difference. An enzymatic activity that produces 1 µmol of hydroxamic acid per minute was defined as 1 U.

### <2> Introduction of mutation into TG

Mutation was introduced into TG by using pPSPTG1 (Appl. Environ. Microbiol., 2003, 69(1), 358-366), which is an expression plasmid for TG, as a template and using PrimeSTAR(R) Max DNA Polymerase (Takara Bio). pPSPTG1 is a secretory expression plasmid for TG having a pro-structure moiety of *Streptoverticillium mobaraense.* Two PCR fragments were prepared by using primers shown in Table 1, and the PCR fragments were mutually ligated by using In-Fusion(R) HD Cloning Kit (Takara Bio), to thereby an expression plasmid for TG introduced with an objective mutation. A double mutant was constructed by using the constructed single mutant as a template and introducing an additional mutation via the same procedure. A triple mutant was constructed by using the constructed double mutant as a template and introducing an additional mutation via the same procedure.

**Table 1 Primers used for introduction mutations**

| Mutation | | Fw primer SEQ ID NO | Rv primer SEQ ID NO |
|---|---|---|---|
| M16T | PCR fragment 1 | 5 | 7 |
| | PCR fragment 2 | 8 | 6 |
| Y34F | PCR fragment 1 | 5 | 9 |
| | PCR fragment 2 | 10 | 6 |
| W38F | PCR fragment 1 | 5 | 11 |
| | PCR fragment 2 | 12 | 6 |
| D46P | PCR fragment 1 | 5 | 13 |
| | PCR fragment 2 | 14 | 6 |
| S199A | PCR fragment 1 | 5 | 15 |
| | PCR fragment 2 | 16 | 6 |
| A261C | PCR fragment 1 | 5 | 17 |
| | PCR fragment 2 | 18 | 6 |
| V271C | PCR fragment 1 | 5 | 19 |
| | PCR fragment 2 | 20 | 6 |
| A322C | PCR fragment 1 | 5 | 21 |
| | PCR fragment 2 | 22 | 6 |
| V326C | PCR fragment 1 | 5 | 23 |
| | PCR fragment 2 | 24 | 6 |

### <3> Construction of mutant TG-expressing strains

The expression plasmids for mutant TGs constructed in <2> were each introduced into *Corynebacterium glutamicum* YDK010 (WO 2002/081694 A1) by electroporation. YDK010 is a cell surface layer protein PS2 deficient strain of C. *glutamicum* AJ12036 (FERM BP-734). AJ12036 was originally deposited at the Fermentation Research Institute, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on March 26, 1984 as an international deposit, and assigned an accession number of FERM BP-734. Cells after electroporation were cultured on a CM-Dex plate (5 g/L glucose, 10 g/L polypeptone, 10 g/L Yeast extract, 1 g/L KH₂PO₄, 0.4 g/L MgSO₄-7H₂O, 3 g/L urea, 0.01 g/L FeSO₄-7H₂O, 0.01 g/L MnSO₄-5H₂O, 10 µg/L biotin, 1.2 g/L hydrochloric acid hydrolysate of soybeans (as the total nitrogen), adjusted to pH 7.0 with KOH, and 20 g/L Agar) containing 25 mg/L kanamycin at 30°C. An obtained colony was purified on a CM-Dex plate containing 25 mg/L kanamycin and obtained as a mutant TG-expressing strain. The obtained strain was cultured in 3 mL of a CM-Dex liquid medium containing 25 mg/L kanamycin at 30°C for approximately 16 hours, a 0.6 mL-aliquot of the culture broth was mixed with 0.6 mL of 40% glycerol, and stored as a glycerol stock at -80°C.

### <4> Culturing of mutant TG-expressing strains

A small amount of the glycerol stock of the mutant TG-expressing strain obtained in <3> was scraped off, applied on a CM-Dex plate containing 25 mg/L kanamycin, and cultured at 20°C for 3 days. Cells grown on the plate were inoculated in 10 mL of a CM2G medium (5 g/L glucose, 10 g/L polypeptone, 10 g/L Yeast extract, 5 g/L NaCl, 0.2 g/L DL-methionine, adjusted to pH 7.0 with KOH) containing 25 mg/L kanamycin, and cultured using a large test tube at 30°C for 24 hours with shaking. A 2.5 mL-aliquot of the obtained culture broth was inoculated in 50 mL of a TG production medium (60 g/L glucose, 1 g/L MgSO₄-7H₂O, 0.01 g/L FeSO₄-7H₂O, 0.01 g/L MnSO₄-5H₂O, 30 g/L(NH₄)₂SO₄, 1.5 g/L KH₂PO₄, 0.15 g/L DL-methionine, 0.45 mg/L thiamine hydrochloride, 0.45 mg/L Biotin, adjusted to pH 7.5 with KOH) containing 25 mg/L kanamycin and 50 g/L CaCO₃, and cultured using a shake flask at 30°C for 48 hours. For A322C-expressing strain, V271C/V326C-expressing strain, A261C/A322C-expressing strain, and A261C/A322C/S199A-expressing strain, DTT was added at a final concentration of 3 mM 5 hours after the start of culture. After completion of culture, the culture broth was collected in a plastic bottle, and stored at - 80°C.

### <5> Purification of mutant TGs

The culture broth obtained in <4> was thawed, centrifuged (8000 rpm, 4°C, 20 min), and filtered through a 0.45 µm filter, to obtain a sterilized solution. The sterilized solution was subject to solvent exchange with 20 mM acetate buffer (pH 5.5) at room temperature by using Sephadex G25(M) (GE Healthcare). After adjustment to pH 7.0 with NaOH, Alcalase (Sigma-Aldrich, P4860-50ML) was added at 0.5% by weight to the mutant TG, and the reaction was carried out at 30°C for 17 hours, to activate the mutant TG. After completion of the reaction, the resulting solution was adjusted to pH 5.5 with 10% acetic acid, and the whole quantity thereof was applied to a cation exchange column (Resource S 6 mL, GE Healthcare) well equilibrated with 20 mM acetic acid buffer (pH 5.5). After re-equilibration with the same buffer, elution was carried out with a linear concentration gradient of 0 to 0.5 M NaCl, and protein fractions eluted at around 200 mM of NaCl were fractionated on the basis of UV absorption at a wavelength of 280 nm as an indicator. The TG activity and TG amount of each fraction were measured by the method described in <1>, and fractions near the peak top having almost equal specific activity, excluding fractions having low specific activity, were collected. The collected fractions were subject to solvent exchange with 20 mM phosphate buffer (pH 6.0) by using HiPrep 26/10 Desalting (GE Healthcare) and used as the mutant TG in the following experiments. Both cation exchange chromatography and buffer exchange were carried out at 4°C.

### <6> Evaluation of specific activity of mutant TGs

The mutant TG obtained in <5> was diluted with 20 mM MES buffer (pH 6.0) to 0.05 mg/mL in terms of BSA. The TG activity of the diluted solution was measured by the method described in <1>, to calculate the specific activity of the mutant TG. As a control, a wild-type TG (prepared from Activa(R)) was analyzed via the same procedure. The results are shown in Table 2.

**Table 2 Specific activities of mutant TGs**

| Mutation | Specific activity (U/mg) |
|---|---|
| Wild-type | 28.4 |
| W38F | 31.4 |
| D46P | 27.4 |
| S199A | 33.2 |
| M16T/S199A | 40.8 |
| M16T/Y34F | 32.0 |
| V271C/V326C | 36.0 |
| A322C | 25.7 |
| A261C/A322C | 30.3 |
| A261C/A322C/S199A | 37.9 |

### <7> Evaluation of acid tolerance of mutant TGs

The mutant TG obtained in <5> was diluted with 20 mM sodium acetate buffer (pH 4.0, 4.5, 5.0, or 5.5) or 20 mM MES buffer (pH 6.0) to 0.02 mg/mL in terms of BSA, and treated at 37°C for one hour. The residual TG activity after treatment at each pH was measured by the hydroxamate method. Solution A (50 mM MES, 100 mM NH₂OH, 10 mM glutathione (reduced form), 30 mM CBZ-Gln-Gly (Z-QG), adjusted to pH 6.0 with NaOH) (500 µL) was added in a 1.5 mL tube, and heated at 37°C for 5 min. After 150 µL of an enzyme solution after treatment at each pH was added and the reaction was carried out at 37°C for 30 min, 500 µL of solution B (1N HCl, 4% TCA, 1.67% FeCl₃-6H₂O) was added to stop the reaction. A reaction stop solution (200 µL) was added to a 96-well plate, and the absorbance at 525 nm was measured using a plate reader. As a control, the absorbance was measured for a resulting mixture similarly obtained by a reaction using 20 mM MES pH 6.0, and the absorbance difference from the sample solution was determined. The absorbance difference in the sample after treatment at each pH was calculated as a relative value to the absorbance difference in the sample after treatment at pH 6.0 which was taken as 100%. This relative value of the absorbance difference was considered as a relative value of the residual TG activity after treatment at each pH to the residual TG activity after treatment at pH 6.0 which was taken as 100%. As a control, a wild-type TG (prepared from Activa(R)) was analyzed via the same procedure. The results are shown in Table 3. A decrease in the residual TG activity due to acid treatment was observed for the mutant TGs, whereas no decrease in the residual TG activity due to acid treatment was observed for the wild-type TG. Hence, it was revealed that the acid tolerance has been lowered in the mutant TGs.

**Table 3 Relative values of residual TG activity after treatment at respective pH**

| | pH 4.0 | pH 4.5 | pH 5.0 | pH 5.5 | pH 6.0 |
|---|---|---|---|---|---|
| Wild-type | 96% | 104% | 106% | 104% | 100% |
| W38F | 16% | 75% | 64% | 102% | 100% |
| D46P | 8% | 16% | 28% | 79% | 100% |
| S199A | 50% | 90% | 94% | 96% | 100% |
| M16T/S199A | 14% | 66% | 84% | 101% | 100% |
| M16T/Y34F | 24% | 77% | 89% | 98% | 100% |
| V271C/V326C | 32% | 53% | 55% | 84% | 100% |
| A322C | 20% | 69% | 65% | 91% | 100% |
| A261C/A322C | 7% | 41% | 43% | 93% | 100% |
| A261C/A322C/S199A | 1% | 12% | 22% | 86% | 100% |

### Example 2: Preparation and analysis of yogurt using mutant transglutaminases (mutant TGs)

### <1> Preparation of yogurt

Pasteurized milk, water, and raw heat powder were mixed to prepare ingredient-adjusted milk (protein: 3.8 wt%, lipid: 1.0 wt%). The prepared ingredient-adjusted milk was heated at 95°C for 3 min, cooled to 43°C, and dispensed in 200 g portions. One mL of a lactic acid bacterium (Nozawagumi, YF-L811 lactic acid bacteria starter) dispersed in the ingredient-adjusted milk was added to the dispensed ingredient-adjusted milk at a final concentration of 0.006%. In addition, a wild-type TG (prepared from the pPSPTG1-expressing strain) or the mutant TG obtained in <5> of Example 1 (W38F, S199A, M16T/A199A, M16T/Y34F, V271C/V326C, or A261C/A322C) was added to the dispensed ingredient-adjusted milk at 1 U per 1 g of the milk protein. After addition, the resulting mixture was promptly divided into approximately 30 mL portions, and incubated at 43°C for approximately 6 hours until the pH decreased to 4.6 or lower. After incubation, the pH of the fermentation product was confirmed to be 4.5 to 4.6, and the fermentation product was further cooled at 5°C overnight.

### <2> Measurement of breaking strength of yogurt

It is known that the reaction of TG forms covalent bonds between lysine or glutamine residues, which increases the breaking strength of yogurt. Hence, the breaking strength of the prepared yogurt was measured with a texture analyzer as an indicator of the reactivity of each TG. The analysis conditions are shown below. Four subdivided samples were used per sample, to carry out the analysis with n=4.

Blunger used: Cylinder type (1 cm in diameter)
Return Distance: 50 mm
Return Speed: 10 mm/sec
Contact Force: 0.5 g
Test Mode: Compression
Pre-test speed: 0.5 mm/sec
Test speed: 0.5 mm/sex
Post test speed: 10 mm/sec
Target mode: Strain
Strain: 30%
Trigger type: Auto
Trigger force: 0.5 g
Advanced option: OFF

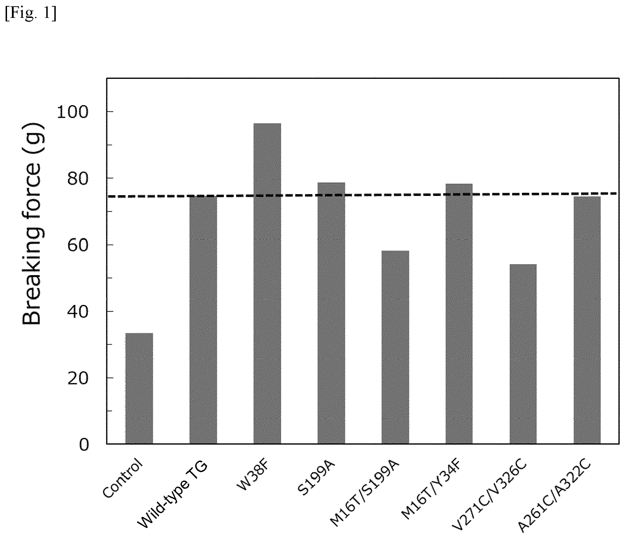

The analysis results are shown in Figure 1. All mutant TGs exhibited an action of improving the breaking strength almost equivalent to that of the wild-type TG.

### <3> Measurement of TG activity in yogurt (hydroxamate method)

The TG activity in the prepared yogurt was measured by the hydroxamate method according to the following procedure.

The prepared yogurt was centrifuged under conditions of 5°C, 10000 g for 30 min, to obtain a supernatant. The obtained supernatant was concentrated by ultrafiltration with a UF membrane (Amicon(R) Ultra15 10k) under conditions of 5°C, 1000 g for 40 min, to obtain an approximately 10-fold concentrated enzyme solution. The concentration factor of each concentrated enzyme solution was calculated from the weight of the solution before and after concentration.

Tris 12.1 g, hydroxylammonium chloride 3.5 g, glutathione (reduced form) 1.55 g, and CBZ-Gln-Gly 5.05 g were added to 500 mL of distilled water, and the pH was adjusted to 6.0 with 3 M hydrochloric acid, to prepare a substrate solution A. 3 M hydrochloric acid, 12% trichloroacetic acid, and 5% ferric chloride in equal volumes were mixed, to prepare a reaction stop solution B. A 350 µL-aliquot of the solution A was added to a 1.5 mL tube and heated at 37°C for approximately 5 min. A 35 µL-aliquot of each concentrated enzyme solution was added to the solution A, the reaction was carried out for 120 or 240 min at 37°C, and then 350 µL of the solution B was added to stop the reaction. A 200 µL-aliquot of the reaction solution after stopping the reaction was added to a 96-well plate, and the absorbance at 525 nm was measured with a plate reader.

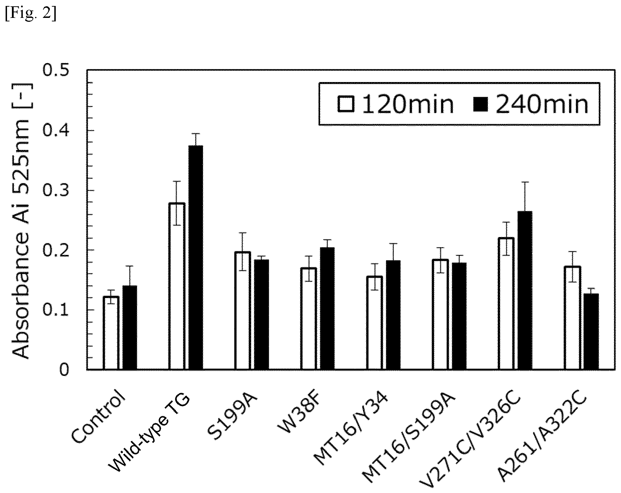

The measurement results are shown in Figure 2. The absorbance of each sample has been corrected to the absorbance at 10-fold concentration on the basis of each concentration factor. All mutant TGs showed lower absorbance than the wild-type TG. Hence, all mutant TGs were considered to have a lower residual activity than the wild-type TG. Furthermore, A261C/A322C showed absorbance almost equivalent to that of the control. Hence, the residual activity of A261C/A322C was considered to be almost zero.

### <4> Measurement of TG activity in yogurt (fluorescence method)

Since A261C/A322C was the most promising in terms of deactivation in the product in Example 2 <3>, the TG activity was measured for A261C/A322C and the wild-type TG by the fluorescence method. A yogurt supernatant was concentrated approximately 15-fold by ultrafiltration with a UF membrane in the same manner as in Example 2 <2>, to obtain a concentrated enzyme solution. A 50 µL-aliquot of the concentrated enzyme solution was added to 200 µL of a substrate solution (79mM Tris-HCl buffer, 150µM Monodancyl cadaverin, 0.5M DTT, and 1 wt% N,N-dimethyl casein), and fluorescence spectra were measured at λ_{EX} 350 nm and λ_{EM} 480 nm for a reaction time of 80 min.

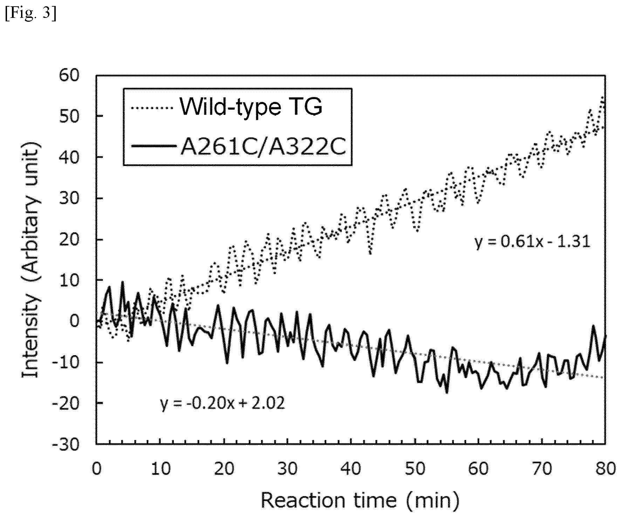

Changes in the fluorescence intensity over time are shown in Figure 3. An increase in fluorescence was observed for the wild-type TG, whereas no increase in fluorescence was observed for A261C/A322C. Hence, the wild-type TG was considered to have a residual activity, whereas A261C/A322C was considered to have no residual activity.

From Example 2 <2> to <4>, it was revealed that A261C/A322C has an action of modifying yogurt almost equivalent to that of the wild-type TG, and is deactivated in the product, to show no residual activity.

### <5> Measurement of pH and TG activity during incubation

Yogurt was prepared in the same manner as in Example 2 <1>, except that the wild-type TG or A261C/A322C was added at 3 U per 1 g of the milk protein. After addition of the lactic acid bacterium, fermentation was started in an incubator at 43°C. Samples were collected every 1.5 hours, starting at the time of addition of the lactic acid bacterium, subject to pH measurement, and then stored in ice-cold conditions. Fermentation was terminated when the pH of the yogurt fell below 4.6, and the yogurt was stored at 5°C overnight. Since the samples after 4.5 hours of fermentation had solidified, they each were separated with a centrifugal separator at 10,000 g for 30 min, to obtain a supernatant, and the supernatant was concentrated approximately 10-fold by ultrafiltration with a UF membrane under conditions of 10,000 g for 30 min. Since the samples before 4.5 hours of fermentation had not solidified, they were subject to subsequent analysis without filtration or concentration. All samples were subjected to activity measurement by the hydroxamate method for a reaction time of 240 min via the same procedure as Example 2 <3>. For the sample at 3 hours of fermentation, only the pH was measured because it was difficult to make side-by-side comparisons due to the difference in the presence or absence of solidification in each sample.

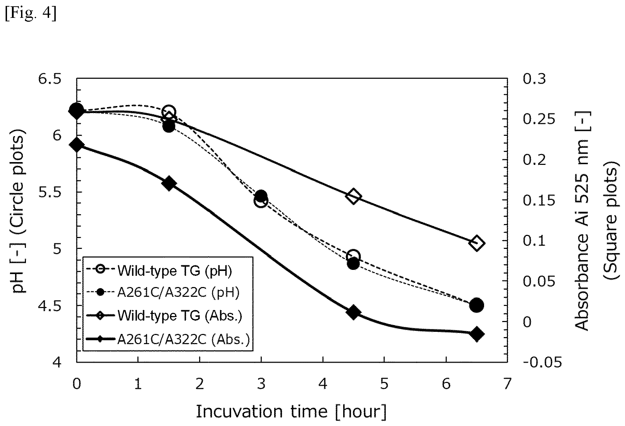

Changes in the pH and the TG activity over time are shown in Figure 4. A261C/A322C showed a greater rate of deactivation accompanying a decrease in the pH during fermentation compared to the wild-type TG. In particular, the wild-type TG showed absorption even at pH 4.6 or lower and had a residual activity, while A261C/A322C showed no absorption at pH 4.6 or lower and was considered to be completely deactivated in the product.

### Industrial Applicability

According to the present invention, a mutant TG having low acid tolerance can be provided. The mutant TG having low acid tolerance can be useful for, for example, modification or production of a food such as yogurt.

### <Explanation of Sequence Listing>

SEQ ID NOS:
1: Nucleotide sequence of a part of the TG gene of *Streptoverticillium mobaraense,* the part encoding the mature TG
2: Amino acid sequence of the mature TG of *Streptoverticillium mobaraense*
3: Nucleotide sequence of the TG gene of *Streptoverticillium mobaraense*
4: Amino acid sequence of TG of *Streptoverticillium mobaraense* comprising the pro-structure moiety
5 to 24: Primers

## Claims

1. A method for producing a food, the method comprising:
a step of treating a food raw material with a mutant transglutaminase,
wherein the food is a food (A) or (B) shown below:
(A) a food whose production is accompanied by a decrease in pH;
(B) a food containing the food (A),
wherein the mutant transglutaminase is a protein having a specific mutation in the amino acid sequence of a wild-type transglutaminase, and having a transglutaminase activity, and
wherein the specific mutation is a mutation for decreasing acid tolerance.

2. The method according to claim 1, wherein the residual activity after treating the mutant transglutaminase at pH4.0, 37°C for one hour is 80% or less in term of the relative value to the residual activity after treating the mutant transglutaminase at pH6.0, 37°C for one hour which is taken as 100%.

3. The method according to claim 1 or 2, wherein the residual activity after treating the mutant transglutaminase at pH4.0, 37°C for one hour is 50% or less in term of the relative value to the residual activity after treating the mutant transglutaminase at pH6.0, 37°C for one hour which is taken as 100%.

4. The method according to any one of claims 1 to 3, wherein the specific mutation is mutation(s) at one or more amino acid residues selected from the followings:
A261, V271, A322, V326, D46, M16, Y34, W38, and S199.

5. The method according to claim 4, wherein the specific mutation comprises mutation(s) at one or more amino acid residues selected from the followings:
A261, V271, A322, V326, and D46.

6. The method according to claim 4 or 5, wherein the specific mutation comprises one or more mutations selected from the followings:
A261C, V271C, A322C, V326C, D46P, M16T, Y34F, W38F, and S199A.

7. The method according to any one of claims 4 to 6, wherein the specific mutation comprises one or more mutations selected from the followings:
A261C, V271C, A322C, V326C, and D46P.

8. The method according to any one of claims 4 to 7, wherein the specific mutation comprises any of the following mutations:
A261C/A322C, A261C/A322C/S199A, V271C/V326C, M16T/Y34F, and M16T/S199A.

9. The method according to any one of claims 1 to 8, wherein the wild-type transglutaminase is a protein having an amino acid sequence of a mature transglutaminase of a *Streptomyces* bacterium.

10. The method according to claim 9, wherein the *Streptomyces* bacterium is *Streptomyces mobaraensis.*

11. The method according to any one of claims 1 to 10, wherein the wild-type transglutaminase is any of the following proteins:
(a) a protein comprising the amino acid sequence of SEQ ID NO: 2;
(b) a protein comprising the amino acid sequence of SEQ ID NO: 2 but including substitution, deletion, insertion, and/or addition of 1 to 10 amino acid residues; and
(c) a protein comprising an amino acid sequence having an identity of 90% or higher to the amino acid sequence of SEQ ID NO: 2.

12. The method according to any one of claims 1 to 11, wherein the food (A) is yogurt or cheese, and wherein the food raw material is a milk raw material.

13. The method according to claim 12, wherein the food (B) is ice cream, the ice cream containing yogurt or cheese.

14. The method according to any one of claims 1 to 13, wherein the mutant transglutaminase is added to the food raw material at a time point when the pH of the food raw material is 5.0 or higher.

15. A composition for producing a food, the composition comprising a mutant transglutaminase,
wherein the food is a food (A) or (B) shown below:
(A) a food whose production is accompanied by a decrease in pH;
(B) a food containing the food (A),
wherein the mutant transglutaminase is a protein having a specific mutation in the amino acid sequence of a wild-type transglutaminase, and having a transglutaminase activity, and
wherein the specific mutation is a mutation for decreasing acid tolerance.

16. The composition according to claim 15, wherein the residual activity after treating the mutant transglutaminase at pH4.0, 37°C for one hour is 80% or less in term of the relative value to the residual activity after treating the mutant transglutaminase at pH6.0, 37°C for one hour which is taken as 100%.

17. The composition according to claim 15 or 16, wherein the residual activity after treating the mutant transglutaminase at pH4.0, 37°C for one hour is 50% or less in term of the relative value to the residual activity after treating the mutant transglutaminase at pH6.0, 37°C for one hour which is taken as 100%.

18. The composition according to any one of claims 15 to 17, wherein the specific mutation is mutation(s) at one or more amino acid residues selected from the followings:
A261, V271, A322, V326, D46, M16, Y34, W38, and S199.

19. The composition according to claim 18, wherein the specific mutation comprises mutation(s) at one or more amino acid residues selected from the followings:
A261, V271, A322, V326, and D46.

20. The composition according to claim 18 or 19, wherein the specific mutation comprises one or more mutations selected from the followings:
A261C, V271C, A322C, V326C, D46P, M16T, Y34F, W38F, and S199A.

21. The composition according to any one of claims 18 to 20, wherein the specific mutation comprises one or more mutations selected from the followings:
A261C, V271C, A322C, V326C, and D46P.

22. The composition according to any one of claims 18 to 21, wherein the specific mutation comprises any of the following mutations:
A261C/A322C, A261C/A322C/S199A, V271C/V326C, M16T/Y34F, and M16T/S 199A.

23. The composition according to any one of claims 15 to 22, wherein the wild-type transglutaminase is a protein having an amino acid sequence of a mature transglutaminase of a *Streptomyces* bacterium.

24. The composition according to claim 23, wherein the *Streptomyces* bacterium is *Streptomyces mobaraensis.*

25. The composition according to any one of claims 15 to 24, wherein the wild-type transglutaminase is any of the following proteins:
(a) a protein comprising the amino acid sequence of SEQ ID NO: 2;
(b) a protein comprising the amino acid sequence of SEQ ID NO: 2 but including substitution, deletion, insertion, and/or addition of 1 to 10 amino acid residues; and
(c) a protein comprising an amino acid sequence having an identity of 90% or higher to the amino acid sequence of SEQ ID NO: 2.

26. The composition according to any one of claims 15 to 25, wherein the food (A) is yogurt or cheese, and wherein the food raw material is a milk raw material.

27. The composition according to claim 26, wherein the food (B) is ice cream, the ice cream containing yogurt or cheese.

28. A mutant transglutaminase, having a specific mutation in the amino acid sequence of a wild-type transglutaminase, and having a transglutaminase activity,
wherein the specific mutation is a mutation for decreasing acid tolerance, and
wherein the specific mutation is mutation(s) at one or more amino acid residues selected from the followings:
A261, V271, A322, V326, D46, M16, Y34, W38, and S199,
provided that none of the mutations at M16, Y34, W38, and S199 is selected solely, and
provided that the mutation at D46 is a mutation other than D46C when the mutation at D46 is selected solely.

29. The mutant transglutaminase according to claim 28, wherein the residual activity after treating the mutant transglutaminase at pH4.0, 37°C for one hour is 80% or less in term of the relative value to the residual activity after treating the mutant transglutaminase at pH6.0, 37°C for one hour which is taken as 100%.

30. The mutant transglutaminase according to claim 28 or 29, wherein the residual activity after treating the mutant transglutaminase at pH4.0, 37°C for one hour is 50% or less in term of the relative value to the residual activity after treating the mutant transglutaminase at pH6.0, 37°C for one hour which is taken as 100%.

31. The mutant transglutaminase according to any one of claims 28 to 30, wherein the specific mutation comprises mutation(s) at one or more amino acid residues selected from the followings:
A261, V271, A322, V326, and D46.

32. The mutant transglutaminase according to claim 31, wherein the specific mutation comprises one or more mutations selected from the followings:
A261C, V271C, A322C, V326C, D46P, M16T, Y34F, W38F, and S199A.

33. The mutant transglutaminase according to claim 31 or 32, wherein the specific mutation comprises one or more mutations selected from the followings:
A261C, V271C, A322C, V326C, and D46P.

34. The mutant transglutaminase according to any one of claims 31 to 33, wherein the specific mutation comprises any of the following mutations:
A261C/A322C, A261C/A322C/S199A, V271C/V326C, M16T/Y34F, and M16T/S199A.

35. The mutant transglutaminase according to any one of claims 28 to 34, wherein the wild-type transglutaminase is a protein having an amino acid sequence of a mature transglutaminase of a *Streptomyces* bacterium.

36. The mutant transglutaminase according to claim 35, wherein the *Streptomyces* bacterium is *Streptomyces mobaraensis.*

37. The mutant transglutaminase according to any one of claims 28 to 36, wherein the wild-type transglutaminase is any of the following proteins:
(a) a protein comprising the amino acid sequence of SEQ ID NO: 2;
(b) a protein comprising the amino acid sequence of SEQ ID NO: 2 but including substitution, deletion, insertion, and/or addition of 1 to 10 amino acid residues; and
(c) a protein comprising an amino acid sequence having an identity of 90% or higher to the amino acid sequence of SEQ ID NO: 2.

38. A gene encoding the mutant transglutaminase according to any one of claims 28 to 37.

39. A vector comprising the gene according to claim 38.

40. A microorganism having the gene according to claim 38.

41. The microorganism according to claim 40, which is a bacterium or a yeast.

42. The microorganism according to claim 40 or 41, which is a coryneform bacterium or a bacterium of the family *Enterobacteriaceae.*

43. The microorganism according to any one of claims 40 to 42, which is a *Corynebacterium* bacterium or an *Escherichia* bacterium.

44. The microorganism according to any one of claims 40 to 43, which is *Corynebacterium glutamicum* or *Escherichia coli.*
